(19) Europäisches Patentamt / European Patent Office / Office européen des brevets

(11) **EP 2 178 577 B1**

(12) **EUROPEAN PATENT SPECIFICATION**

(45) Date of publication and mention
of the grant of the patent:
**10.09.2014 Bulletin 2014/37**

(21) Application number: **08796142.1**

(22) Date of filing: **10.07.2008**

(51) Int Cl.:
*A61L 27/50* (2006.01)   *G01N 27/00* (2006.01)

(86) International application number:
**PCT/US2008/069654**

(87) International publication number:
**WO 2009/009666 (15.01.2009 Gazette 2009/03)**

(54) **METHOD TO ENHANCE OSTEOBLAST FUNCTIONALITY AND MEASURE ELECTROCHEMICAL PROPERTIES FOR A MEDICAL IMPLANT**

VERFAHREN ZUR ERWEITERUNG VON OSTEOBLAST-FUNKTIONEN UND ZUR MESSUNG DER ELEKTROCHEMISCHEN EIGENSCHAFTEN EINES MEDIZINISCHEN IMPLANTATS

PROCÉDÉ PERMETTANT D'ACCROÎTRE LA FONCTION OSTÉOBLASTIQUE ET DE MESURER LES PROPRIÉTÉS ÉLECTROCHIMIQUES D'UN IMPLANT MÉDICAL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.07.2007  US 949373 P**
**12.07.2007  US 949386 P**

(43) Date of publication of application:
**28.04.2010  Bulletin 2010/17**

(73) Proprietor: **Nanovis, Inc**
**Columbia City, IN 46725 (US)**

(72) Inventors:
• **SIRIVISOOT, Sirinrath**
**Providence, RI 02912-9104 (US)**
• **YAO, Chang**
**Providence, RI 02912-9104 (US)**
• **XIAO, Xingcheng**
**Troy, MI 48098-2000 (US)**

• **SHELDON, Brian**
**Providence, RI 02912-9104 (US)**
• **WEBSTER, Thomas**
**Providence, RI 02912-9104 (US)**

(74) Representative: **Thom, Russell et al**
**Murgitroyd & Company**
**Scotland House**
**165-169 Scotland Street**
**Glasgow G5 8PL (GB)**

(56) References cited:
WO-A1-01/87193    WO-A1-2004/052447
WO-A2-2006/004686    WO-A2-2006/104644
WO-A2-2006/116752    US-A1- 2005 038 498

• **Oh S, Daraio C, Chen LH, Pisanic TR, Fiñones RR, Jin S.: "Significantly accelerated osteoblast cell growth on aligned TiO2 nanotubes" J Biomed Mater Res A vol. 78A, 6 April 2006 (2006-04-06), pages 97-103, XP002560511 DOI: 10.1002/jbm.a. 30722 [retrieved on 2009-12-10]**

**Description**

Technical Field

This invention relates, in general, to a medical implant having enhanced cytocompatibility capabilities, and is particular to a method of manufacturing a medical implant with a biosensor comprising treating the surface of a medical implant to increase osteoblast functionality and enhance its electrochemical properties.

**Background of the Invention**

Bone matrices are generally ninety percent (90%) by weight nanostructured fibrillar type-I collagen and ten percent (10%) by weight nanostructured hydroxyapatite crystals. Osteoblasts form the nanostructured organic matrix of bone and produce alkaline phosphatase as well as other proteins which play critical roles in the mineralization process. Undoubtedly, medical implants require the functions of osteoblasts to create new bone on their surfaces; the lack of sufficient new bone growth on current materials have contributed in part to current average hip implant lifetimes of less than fifteen (15) years. In fact, although due to numerous psychological and physical reasons, a recent University of Maryland Medical School (USA) study reported that up to twenty-nine percent (29%) of patients receiving a hip implant die in the following revision surgery. Such data clearly indicate that much is needed to improve the performance of current bone implants.

Surprisingly, no gold standard material exists for orthopedic applications; commercially pure titanium (Ti), cobalt-chromium alloy (CoCrMo), and a titanium alloy (Ti6Al4V) are common variants each with varying degrees of success towards promoting new bone growth. Ti is well-known for its high strength-to-weight ratio, low toxicity, and consequently is the most widely utilized material in orthopedic and maxillofacial replacements. Not only are the mechanical properties of Ti (such as stiffness, high load resistance, fatigue resistance and ductility) sufficient for physiological loading, but its biocompatibility properties are also attractive for orthopedic applications. Important in the design of successful implants is the ability of such materials to control protein adsorption and consequently osteoblast adhesion after they are implanted. The degree to which proteins absorb on implant surfaces depends on biomaterial properties, such as their chemistry, charge, wettability, and topography. In the case of surface chemistry, oxidized layers of titanium oxide ($TiO_2$) are formed on Ti surfaces simply through their exposure to air and/or water. After implantation, oxidized Ti surfaces bind with water, forming $-O^-$ and $-OH^-$ sites which possess a weak negative charge at physiological pH. Therefore, this oxidized layer provides a kinetic barrier that prevents Ti from corroding and provides bone implant materials that promote calcium phosphate crystal, protein, and cellular bonding.

Ti can be improved for orthopedic applications. Resulting changes in topography from Ti oxidation can be modified in order to increase biologically inspired nanometer surface roughness for better protein adsorption, osteoblast attachment, and eventual osseointegration. Recent research has shown nanometer surfaces of anodized Ti may be created to enhance osteoblast adhesion, wherein anodized Ti creates nanotube-like pores, which, have been shown to possess higher surface energy and improved wettability compared to unanodized Ti.

Although improving bone formation appears to be achievable, the clinical diagnosis of new bone growth or identifying other tissue formation surrounding implants (such as through X-rays, magnetic resonance imaging, or bone scans) remains problematic, sometimes significantly increasing patient hospital stay and decreasing the ability to quickly prescribe a change in action if new bone growth is not occurring surrounding the implant. Specifically, the current state of the art for determining whether any tissue in growth has occurred at the implant-tissue interface is for the clinician to perform a physical examination, for example, palpation, or laboratory testing might be completed before imaging techniques are used to inform a clinician about a patient's health. Although advanced imaging techniques, such as bone scans, computer tomography scans, and radiographs (X-rays) are important in medical diagnosis, each has its own limitations and difficulties. A bone scan is used to identify areas of abnormal active bone formation, such as arthritis, infection, or bone cancer. However, bone scans require an injection of a radioactive substance (e.g., technetium) and a prolonged delay for absorbance before the scan can be performed. A computer tomography combines X-rays with computer technology to produce a two dimensional cross-sectional image of a body on the computer screen. Although this technique produces more detail that an X-ray in some cases (e.g., severe trauma to the chest, abdomen, pelvis or spinal cord), a dye (e.g. barium sulfate) must be injected in order to improve the clarity of the image. This often causes pain to the patient

Another evaluation technique, called electromyography, has also been used to analyze/diagnose nerve functions inside body conditions. Thin electrodes are placed in soft tissues to help analyze and record electrical activity in the muscles. However, this electrode technique leads to pain and discomfort for the patient When these needles are removed, soreness and bruising can occur. In contrast, the disclosed inventive electrochemical biosensors on the implant itself will be able to provide *in situ* medical diagnostics and will to likely determine new bone growth surrounding the implant.

Thus, a longstanding need has existed for development of an electrochemical biosensor that is capable of providing specific quantitative or semi-quantitative information using a biological recognition element retained in direct spatial contact with an electrochemical transduction element. The electrochemical biosensor could translate information from the biochemical domain into an electrical output signal to be detected, leading to enhanced understanding of biological functions, including osseointegration or the identification of the type of tissue formation. A further need existed in developing a method of fully integrating a biosensor with a medical implant, or more specifically an orthopaedic implant to allow a clinician to monitor implant-tissue interfaces.

The prior art discloses several nanostructures including medical devices having nanostructures. Relevant documents are WO2006/116752, WO2006/004686, WO2006/104644, WO01/87193, WO2004/052447, US2005/0038498 and Oh. S et al 'Significantly accelerated osteoblast cell growth on aligned TiO2 nanotubes' J Biomed Mater Res A Vol 78A, 6 April 2001, pages 97-103.

Accordingly, in view of these longstanding deficiencies of current construct materials for medical devices and the corresponding lack of consistent osteoblast activity relative to the surface of a medical implant *in vivo* as well as the ability to measure and identify adjacent tissue formation, it would be desirable to develop a process by which materials from which medical devices are fabricated are treated resulting in enhanced cellular functionality, specifically osteoblast proliferation *in vivo,* in conjunction with creating a mechanism or sensor device for determining and *monitoring in situ* new bone growth or other tissue formation surrounding an implanted medical implant.

**Summary of the Invention**

Enhancement in the functionality of materials that are used to fabricate medical devices is desirable. The present invention provides improvements to the cytocompatibility properties of titanium metal that is to be used in fabricating a medical implant. There is described a method of enhancing and increasing osteoblast functionality of a medical device by obtaining a medical implant and treating a surface of the medical implant to modify the surface characteristics resulting in increased functionality of adjacent osteoblasts.

Also described is a method of increasing cellular activity for a medical implant by obtaining a medical implant and processing the surface of the medical implant to change the surface topography causing increased cellular mineral deposition on the surface by cells that are positioned adjacent to the medical implant surface.

The present invention provides a medical implant that has enhanced cytocompatibility that includes a metallic substrate with an outer surface that includes a myriad of attached nanosized structures, as claimed hereinafter. Preferred embodiments of the invention are set forth in the dependent claims.

The present invention also provides a biosensor with the medical implant. The biosensor has an electrode that is attached to the medical implant's outer surface allowing the biosensor to detect electrochemical changes to for identify the presence and type of adjacent tissue. It would be contemplated that such types of tissue including, but are not limited to, bone, soft, connective, including collagen, tendon, cartilage and other biological precursors of these tissue types.

The present invention also provides in yet another aspect, a method of manufacturing a medical implant with a biosensor for use *in vivo* to monitor electrochemical changes along the interface between living tissue and a medical implant. The method may include obtaining the medical implant and treating the surface of the medical implant to modify the surface characteristics, thus causing the formation of the attached biosensor.

Also described is a method of integrating a biosensor with the medical implant. The method may include the steps of obtaining the medical implant, applying a treatment process to the outer surface of the medical implant, producing a plurality of anodized nanotubular structures on the outer surface with each of the nanotubular structures having a lumen, growing carbon nanotubes within the lumens of the plurality of anodized nanotubular structures. The biosensor being constructed of the plurality of nanotubular structures in combination with the carbon nanotubes.

Further, additional features and advantages are realized through the techniques of the present invention.

**Brief Description of the Drawings**

The invention is further described from the following detailed description taken in conjunction with the accompanying drawings in which:

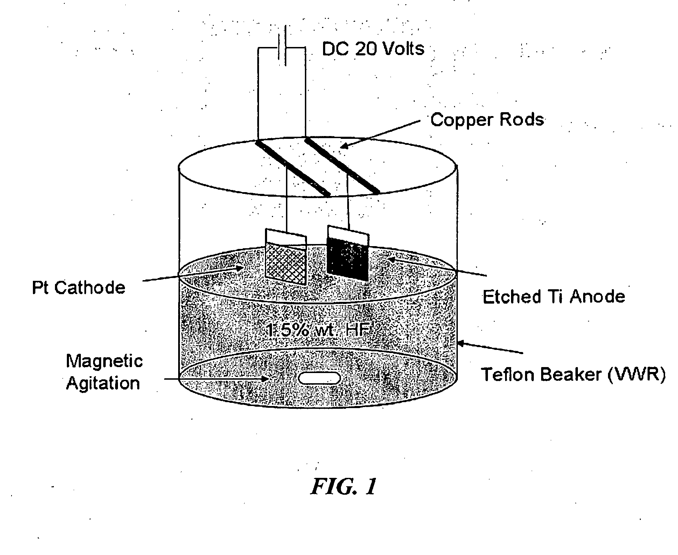

FIG. 1 is a schematic showing the anodization process and vessel in which twenty (20) DC volts were applied for ten (10) minutes in 1.5 wt % hydrofluoric acid to modify the titanium surface to produce nanotubes, in accordance with an aspect of the invention;

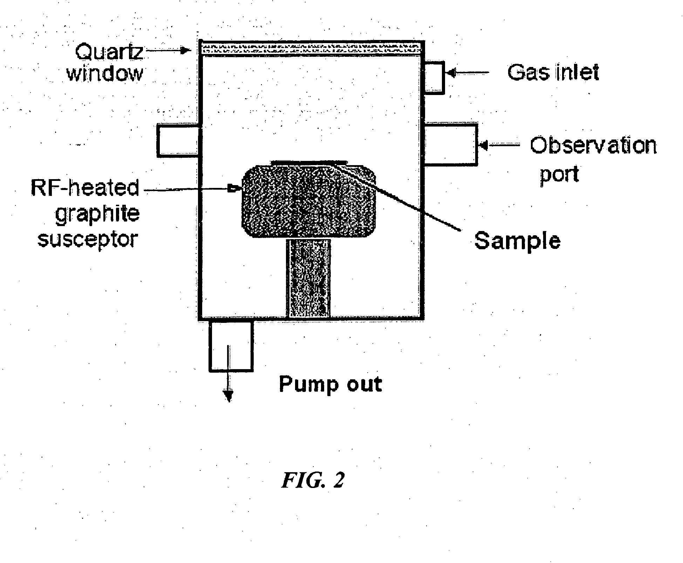

FIG. 2 is a schematic that shows the chemical vapor deposition system used to grow carbon nanotubes out of anodized nanotubular titanium, in accordance with an aspect of the invention;

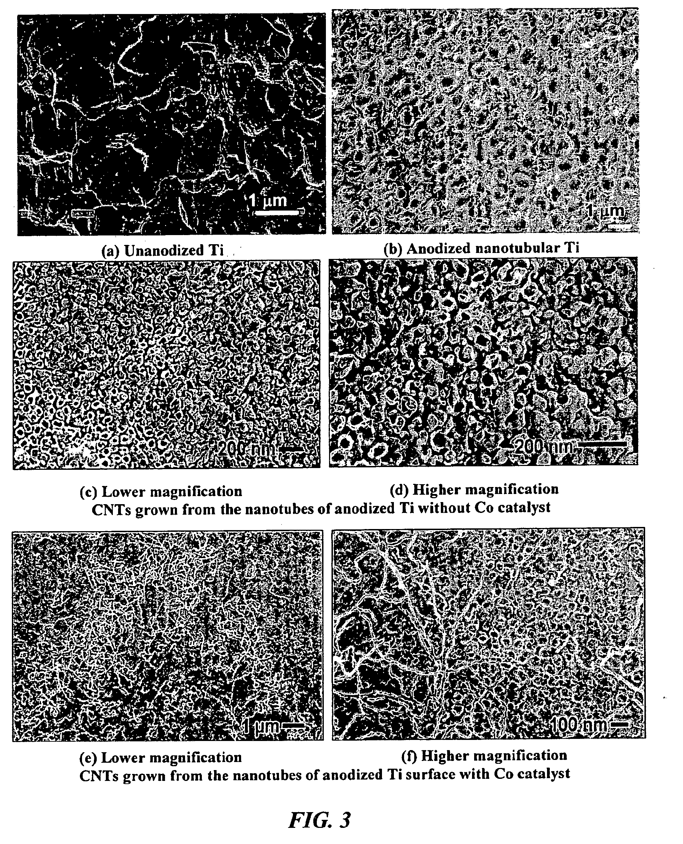

FIGS. 3 (a), (b), (c), (d), (e) and (f) are images of scanning electron microscope micrographs of: (a) unanodized titanium, (b) anodized nanotubular titanium without carbon nanotubes, (c) lower and (d) higher magnification of carbon nanotubes grown from the nanotubes of the anodized titanium without a cobaltous nitrate catalyst; and (e) lower and (f) higher magnification of carbon nanotubes grown from the nanotubes of anodized titanium surface with a cobaltous nitrate catalyst, in accordance with an aspect of the invention;

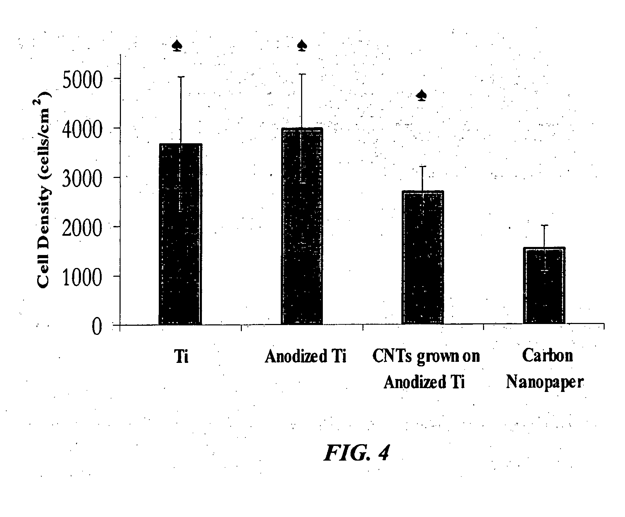

FIG 4. is a bar graph showing the cell density (i.e., osteoblast adhesion) after four (4) hours on unanodized titanium, anodized nanotubular titanium, carbon nanotubes grown on nanotubular anodized titanium, and carbon nanopaper, in accordance with an aspect of the invention;

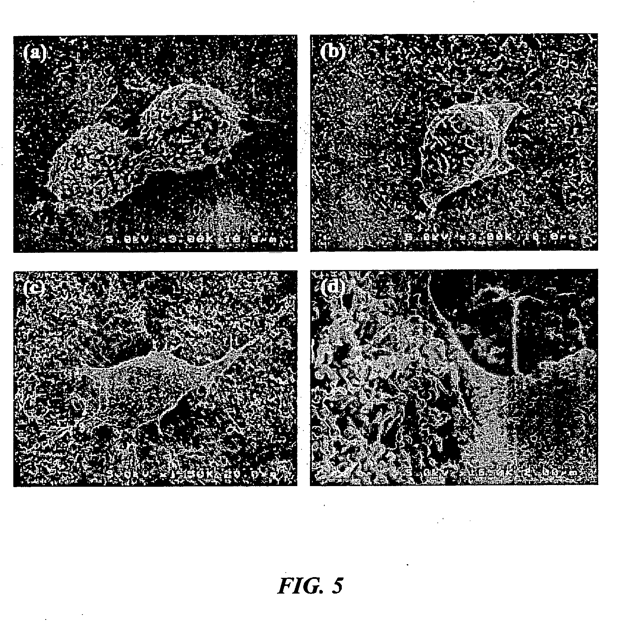

FIGS. 5 (a), (b), (c), and (d) are images of scanning electron microscope micrographs of osteoblast adhesion after four (4) hours on: (a), (b) anodized titanium (scale bars = 10 $\mu$m); and (c), (d) multi-walled carbon nanotubes grown out of anodized nanotubular titanium (scale bars = 20 $\mu$m (c) and 2 $\mu$m (d)), in accordance with an aspect of the invention;

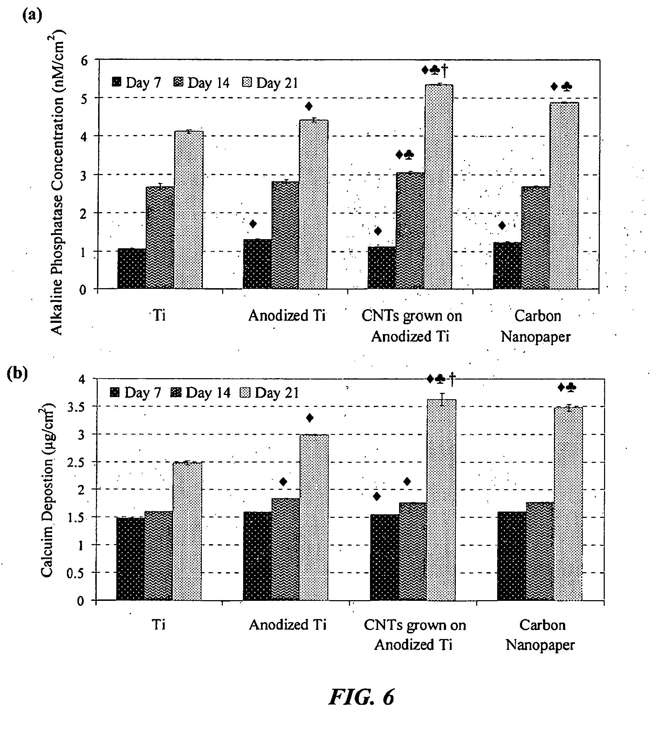

FIGS. 6 (a) and (b) are bar graphs showing long term osteoblast functions of: (a) alkaline phosphatase activity; values are mean $\pm$ S.E.M; n=3; ♦ p<0.05 compared to unanodized titanium, p<0.05 compared to anodized nanotubular titanium, and † p<0.05 compared to carbon nanopaper; and (b) calcium deposition; values are mean $\pm$ S.E.M; n=3; ♦ p<0.05 compared to unanodized titanium, p<0.05 compared to anodized nanotubular titanium, and † p<0.05 compared to carbon nanopaper, in accordance with an aspect of the invention;

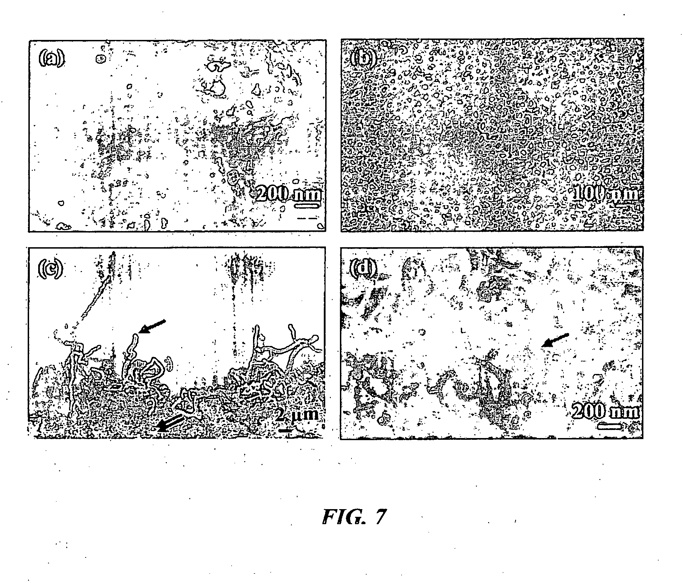

FIGS. 7 (a), (b), (c), and (d) are images of scanning electron microscope micrographs of the electrode surfaces: (a) conventional titanium; (b) anodized titanium; and (c) side view and (d) top view of multi-walled carbon nanotubes-titanium. Single arrow shows multi-walled carbon nanotubes- titanium, whereas the double arrows show the anodized titanium template, in accordance with an aspect of the invention;

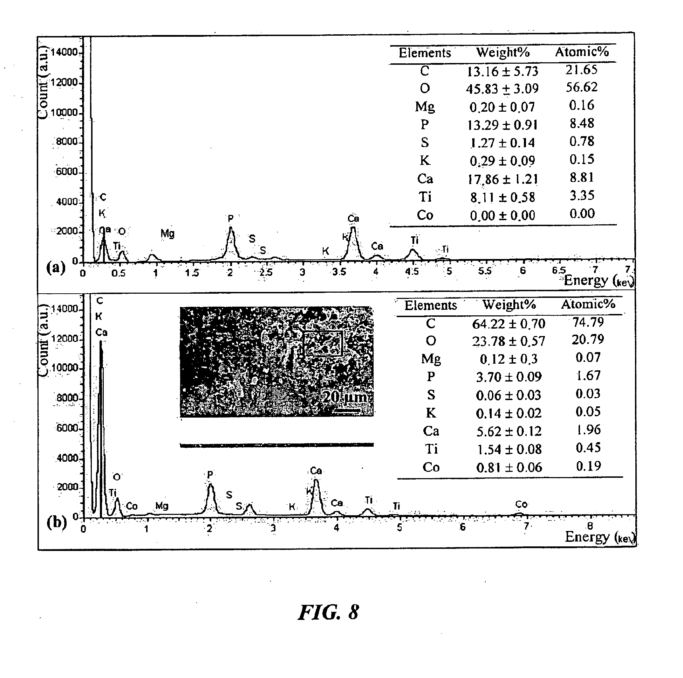

FIGS. 8 (a) and (b) are energy dispersive spectroscopy analysis results of osteoblasts cultured for twenty-one (21) days on: (a) titanium and (b) multi-walled carbon nanotubes- titanium. The scanning electron microscope micrograph of inset (b) shows the analyzed area. For the multi-walled carbon nanotubes- titanium, more Calcium (Ca) and Phosphorous (P) deposited by osteoblasts were observed. Tables in graphs (a) and (b) show the composition of the mineral deposits after osteoblasts were cultured for twenty-one (21) days. The Ca/P weight ratio on bare titanium was 1.32 and on multi-walled carbon nanotubes- titanium was 1.52, in accordance with an aspect of the invention;

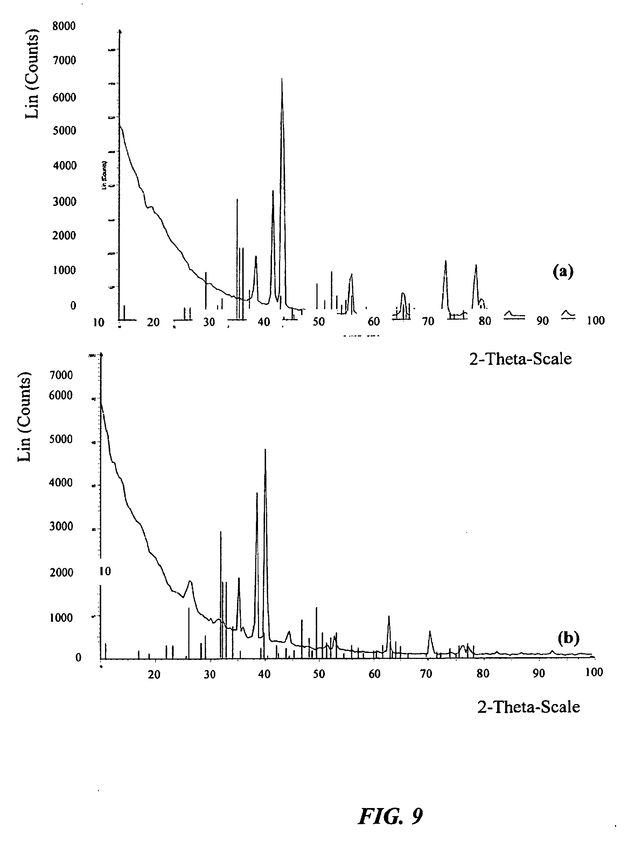

FIGS. 9 (a) and (b) are x-ray diffraction analysis results of hydroxyapatite-like (HA; $Ca_5(PO_4)OH$) deposited minerals after osteoblasts were cultured for twenty-one (21) days on: (a) titanium; and (b) multi-walled carbon nanotubes-titanium. The micrographs show that the peak pattern of HA more closely matches that of the mineral deposited by osteoblasts when cultured on multi-walled carbon nanotubes- titanium than titanium, in accordance with an aspect of the invention;

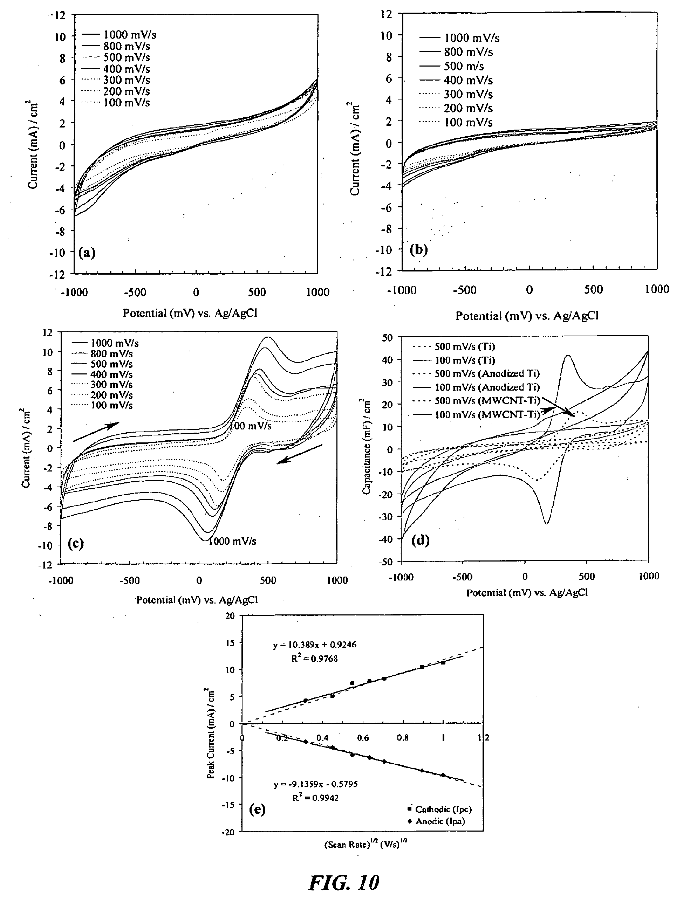

FIGS. 10 (a), (b), and (c) show the results of cyclic voltammograms with an electrolyte solution of 10 mM $K_3Fe(CN)_6$ in 1 M $KNO_3$ for: (a) conventional titanium (commercially pure); (b) anodized titanium; and (c) multi-walled carbon nanotubes-titanium. FIG. 10(d) shows the capacitance of all the electrodes in comparison. FIG. 10(e) shows the plot of the square root of scan rates with anodic peak currents ($I_{pa}$) and cathodic peak currents ($I_{pc}$), in accordance with an aspect of the invention;

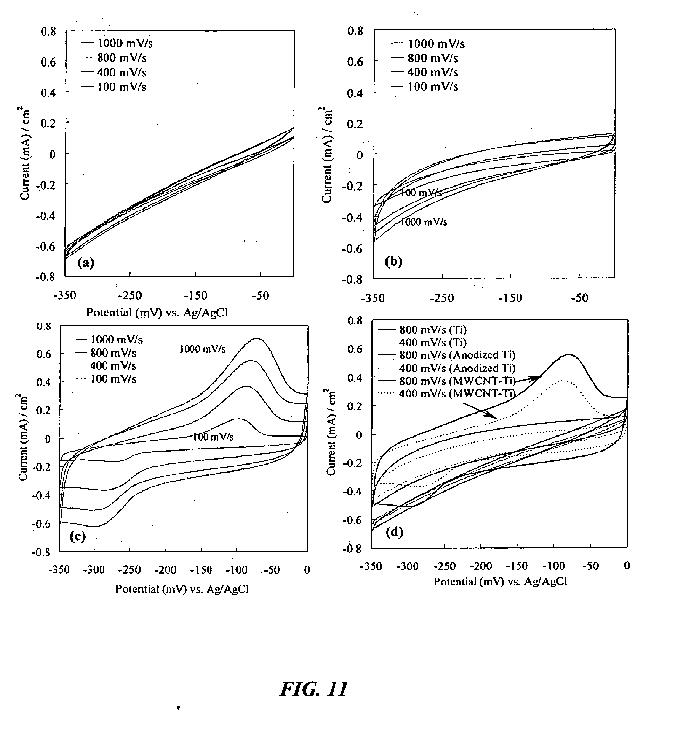

FIGS. 11 (a), (b) and (c) shows the results of cyclic voltammograms with an electrolyte solution of the extracellular matrix secreted by osteoblasts after twenty-one (21) days of culture for: (a) conventional titanium; (b) anodized titanium; and (c) multi-walled carbon nanotubes-titanium with a working area of 1 cm$^2$. FIG. 11(d) shows the cyclic voltammograms of all three electrodes in comparison. Only multi-walled carbon nanotubes-titanium possessed the quasi-reversible redox potential, while conventional titanium and anodized titanium did not, in accordance with an aspect of the invention;

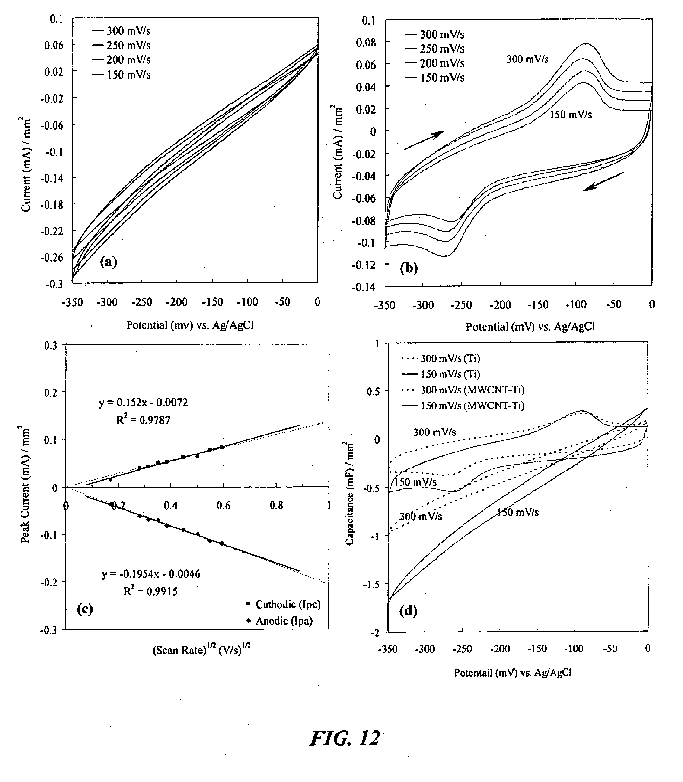

FIGS. 12 (a) and (b) shows the results of cyclic voltammograms with an electrolyte solution of the extra cellular matrix secreted by osteoblasts after twenty-one (21) days of culture for (a) conventional titanium and (b) multi-walled carbon nanotubes-titanium with a working area of 1 mm$^2$, FIG. 12(c) shows a plot of the experimental cathodic and anodic peak currents, obtained from (b), versus the square root of the scan rates; and FIG. 12(d) shows a line graph comparing capacitance of multi-walled carbon nanotubes-titanium and titanium, in accordance with an aspect of the invention; and

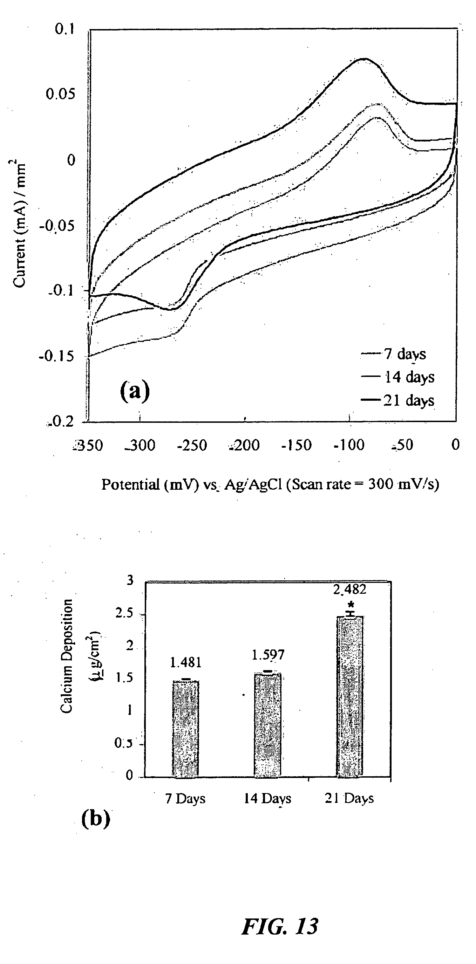

FIG. 13 (a) shows the results of cyclic voltammograms of multi-walled carbon nanotubes-titanium electrodes in an electrolyte solution of the extracellular matrix secreted by osteoblasts cultured on conventional titanium after twenty-one (21) days. FIG. 13(b) shows a bar graph with the results from the calcium deposition assay that determined the calcium concentrations in an electrolyte solution of the extracellular matrix secreted by osteoblasts on conventional titanium after seven (7), fourteen (14), and twenty-one (21) days of culture. Data = mean $\pm$ S.E.M; n=3; *p<0.01 (compared to 7 and 14 days), in accordance with an aspect of the invention.

## Detailed Description of the Invention

The present invention is based in part on the surprising discovery that medical implants that include a surface composed of anodized nanotubular titanium have been shown to have increased osteoblast activity around that medical implant following implantation. Further enhancement of such cytocompatibility is seen when the multi-walled carbon nanotubes are grown on the anodized nanotubular titanium surface. Thus, a process to grow multi-walled carbon nanotubes on the surface of a titanium medical implant that includes a surface of anodized nanotubular structures will result in increased integration with the implant of bone or other types of surrounding tissue including, but are not limited to, soft, connective, including collagen, tendon, cartilage and other biological precursors of these tissue types, that will likely result in longer term implant success. It should be noted that it would be well understood by one skilled in the art that other substrate materials may be used and undergo the subject method for surface topography change and resultant cellular enhancement, with these materials including, but are not being limited to other titanium alloys, cobalt chromium alloys, stainless steel alloys, composites, and polymers.

Accordingly, as disclosed herein is a method for treating a surface of an implant to modify the surface characteristics by growing multi-walled nanotubes, thereby increasing the activity and functionality of adjacent osteoblasts or other cells. Also included is a medical implant on which such process was performed, thus enhancing the cytocompatibility of the medical implant post-implantation.

Also described is the unexpected result that the changed topography of the implant surface creates an integral biosensor on said surface of the medical implant, wherein the conductivity between the biosensor and the surrounding tissue may be measured and allow for tissue identification. The present invention yet further provides for a medical implant to include a self-contained biosensor that is integrally connected to the implant surface and in close approximate to the adjacent/surrounding biological tissue following implantation.

The features and other details of the invention will now be more particularly described with references to the accompanying drawings, experimentation results and claims. Certain terms are defined throughout the specification. Unless otherwise defined, all technical and scientific terms used herein have the same meaning as commonly understood by one of ordinary skill in the art to which this invention pertains. In some cases, terms with commonly understood meanings are defined herein for clarity and/or for ready reference, and the inclusion of such definitions herein should not necessarily be construed to represent a substantial difference over the definition of the term as generally understood in the art. Furthermore, as used herein and in the appended claims, the singular forms include plural referents unless the context clearly dictates otherwise. Thus, for example, reference to "a multi-walled carbon nanotube" includes one or more of such multi-walled carbon nanotubes, as would be known to those skilled in the art.

Discussed below is the novel evaluation undertaken by the inventors that more fully describes the method herein for treating a surface of a titanium medical implant that causes a changed topography and results in enhanced or increased osteoblast functionality and activity, as well as a medical implant that includes an outer surface that has integrally connected anodized nanotubular titanium structures with multi-walled carbon nanotubes growing from within said nanotubular structures.

## *MATERIALS AND METHODS*

### 1. Anodization

In order to create nanotubes on titanium (hereinafter "Ti") for subsequent carbon nanotube tube (hereinafter "CNT") growth, a novel anodization technique was adapted. Briefly, 99.2% commercially pure Ti sheets (Alfa Aesar) were cut into 1x1 cm$^2$ squares and cleaned with acetone, 70% ethanol, and deionized H$_2$O. Then, these samples were etched for 10 seconds with a solution of 1.5% by weight nitric acid and 0.5% by weight hydrofluoric acid to remove the oxidized layer on Ti surfaces. Cleaned Ti samples were used as an anode, while a high purity platinum sheet (Alfa Aesar) served as a cathode. Both were immersed in an electrolyte solution consisting of 1.5% by weight diluted hydrofluoric acid in a Teflon beaker. The surface of the etched Ti was placed next to the platinum sheet at a distance of around 1 cm. As shown in FIG. 1, this anodization system provided twenty (20) volts (DC) for ten (10) minutes to create novel anodized nanotubes on the surfaces of currently-used Ti medical implants.

## 2. Chemical Vapor Deposition

A chemical vapor deposition (hereinafter "CVD") system (Applied Science & Technology Inc.) was used to grow multi-walled CNTs out of anodized Ti nanotubes. First, some of the anodized nanotubular samples were dipped into a 5M cobaltous nitrate solution (Allied Chemical), diluted with methanol to 5 wt %, for 5 minutes to serve as a catalyst for CNT growth. Next, the samples were rinsed with distilled water and dried with compressed air. As depicted in FIG. 2, the samples were placed into a thermal CVD chamber and then air was pumped out to a base pressure below 10 mTorr. The samples were then heated up to 700 °C in a flow of 100 sccm hydrogen gas for 20 minutes. After that, the gas composition was changed to 40 sccm $H_2$ and 160 sccm $C_2H_2$ and applied for 30 minutes to initiate the growth of multi-walled CNTs from the anodized Ti nanotubes. Finally, the samples were cooled in a 100 sccm Ar flow.

All samples were visualized by scanning electron microscopy (SEM; LEO 1530VP FE-4800).

## 3. Cell Culture and Cellular Assays

In vitro osteoblast cytocompatibility assays were determined on four (4) types of samples, including commercially pure Ti, anodized nanotubular Ti, CNTs grown from anodized nanotubular Ti, and carbon nanopaper (buckypaper; NanoLab Inc.). For the CNTs grown from anodized Ti, all samples used in cell assays employed the cobaltous nitrate catalyst. Osteoblast (CRL-11372; American Type Culture Collection) adhesion and differentiation from non-calcium to calcium depositing cells were determined on each substrate. First, all substrates were sterilized by ultraviolet (hereinafter "UV") light exposure for four (4) hours on each side. Substrates were then immediately rinsed with phosphate buffered saline (PBS; 8 g NaCl, 0.2 g KCl, 1.5 g $Na_2PO_4$, and 0.2 g $KH_2PO_4$ in 1000 ml DI water adjusted to a pH of 7.4; Sigma-Aldrich) three times and placed in 12 well plates. For adhesion assays, 3,500 osteoblasts/$cm^2$ were seeded in Dulbecco's Modified Eagle Medium (DMEM) supplemented with 10% fetal bovine serum (FBS; Hyclone), and 1% penicillin/streptomycin (P/S; Hyclone) onto the substrates under standard incubator conditions (a humidified, 5% $CO_2$, and 95% air environment at 37° C) for four (4) hours. At the end of the prescribed time period, cells were then fixed, stained, counted, and visualized by both scanning electron (Leo 1530VP FE-4800) and fluorescence (Lieca) microscopy according to standard techniques.

For osteoblast differentiation assays, 40,000 cells/$cm^2$ were seeded onto the substrates of interest and were cultured in DMEM supplemented with 10% FBS, 1% P/S, 50 nM β-glycerophosphate (Sigma), and 50 μg/ml ascorbic acid (Sigma) under standard incubator conditions for seven (7), fourteen (14), and twenty-one (21) days. At the end of the prescribed time period, an alkaline/acid phosphatase assay kit (Upstate) was used to determine the concentration of alkaline phosphatase in cell lysates. Cell lysates were prepared by first rinsing all samples with Tris-buffered saline (TBS; 42 mM Tris-HCl, 8 mM Tris Base and 0.15 M NaCl; pH of 7.4; Sigma-Aldrich) three times and then subjecting the cells to three freeze-thaw cycles using distilled water. A calcium quantification kit (Sigma) was also used to determine the amount of calcium deposited by osteoblasts cultured on each substrate. An acidic supernatant solution for this assay was prepared by incubating all the samples with 0.6 N HCl (Sigma) overnight. The light absorbance was measured by a spectrophotometer (SpectoMAX; Molecular Devices) at 650 nm for alkaline phosphatase activity and 570 nm for calcium deposition.

All experiments were conducted in triplicate and were repeated at least three (3) times. Analysis of variance (ANOVA) followed by a student t-test was used to determine differences between means.

## *RESULTS*

### 1. Material Surface Topography

As shown in FIG. 3(b), nano-sized tubes were distributed uniformly on the Ti surface following the anodization step of the method. The uniform pores, as observed by scanning electron microscopy, were estimated to have a diameter of 50-60 nm and a depth of 200 nm. Also seen in FIGS. 3(c), (d), (e) and (f) are the parallel multiwalled CNTs successfully grew out of these anodized nanotubes in Ti. Although the topography of anodized nanotubular Ti with CNTs varied between each sample, they were significantly more rough at the nanometer level than both the unanodized and anodized nanotubular Ti without CNTs. Samples pretreated with cobaltous nitrate resulted in more CNT growth from Ti nanotubes than those not pretreated with cobaltous nitrate.

### 2. Osteoblast Responses

As summarized in FIG. 4, followed the performance of the method, similar osteoblast adhesion after four (4) hours on unanodized Ti, anodized nanotubular Ti without CNTs and anodized nanotubular Ti with CNTs; all were greater than the carbon nanopaper.

FIG. 5 shows osteoblasts that were observed to closely interact with CNTs grown out of anodized nanotubular Ti. In contrast, less interactions of osteoblasts with unanodized Ti, anodized Ti , and carbon nanopaper was found.

FIG. 6 depicts the surprising results that following the performance of the disclosed method, alkaline phosphatase activity and calcium deposition by osteoblasts increased on CNTs grown from anodized nanotubular Ti when compared to anodized nanotubular Ti without CNTs, currently used Ti, and carbon nanopaper after twenty-one (21) days.

## *DISCUSSION*

Results of the method confirmed previous work that demonstrated greater osteoblast functions (such as alkaline phosphatase and calcium deposition) on anodized compared to unanodized Ti. Important to note is that anodization is only one of multiple ways to create biologically-inspired nanofeatures on Ti. Similarly, the results of the samples produced by the disclosed method confirmed those of other studies which have demonstrated greater osteoblast functions on carbon nanofibers (hereinafter "CNF") and CNTs compared to currently implantable Ti. It is well known in the art that the novel cytocompatibility properties of CNFs/CNTs direct bone formation to match that of the natural anisotropic arrangement of collagen and hydroxyapatite in long bones of the body.

This evaluation surprisingly advanced the results observed separately on anodized nanotubular Ti and CNTs and provided direct evidence of increased osteoblast activity on CNTs grown from anodized Ti. Importantly, this evaluation provided evidence that while osteoblast adhesion was similar on all substrates tested, markers of osteoblast differentiation (specifically, alkaline phosphatase activity and calcium deposition) were significantly higher when osteoblasts were cultured on CNTs grown from anodized Ti. As mentioned, CNTs have also been used in several sensor applications.

## *CONCLUSION*

The results of the evaluation performed by the inventors and described herein provided the surprising evidence that osteoblasts synthesized more alkaline phosphatase and calcium on the surfaces of non-functionalized CNTs grown from anodized nanotubular Ti compared to anodized nanotubular Ti without CNTs and currently-used unanodized commercially pure Ti. Therefore, such materials can be useful for additional orthopedic and other medical applications, including those in which such nanostructures may serve *as in situ* biosensors monitoring and controlling new bone growth. The inventor's evaluation also demonstrates potential enhanced in vitro bone formation with protruding CNTs for orthopedic applications.

As discussed herein was the ability to address the longstanding need that exists for developing an electrochemical biosensor that is capable of providing specific quantitative or semi-quantitative information using a biological recognition element retained in direct spatial contact with an electrochemical transduction element. The electrochemical biosensor would then translate information from the biochemical domain into an electrical output signal to be detected, leading to enhanced understanding of biological functions, including osseointegration or the identification of other tissue formation. A further need existed in developing a method of fully integrating a biosensor with a medical implant, or more specifically an orthopaedic implant to allow clinician monitoring of implant interfaces.

Accordingly, the present invention provides in another aspect, a self-contained biosensor that is configured to have contact with an implant surface and surrounding tissue following implantation into a patient and measures the conductivity between implant surface and the surrounding tissue, thus providing a means for identify the presence and type of adjacent tissue.

The present invention provides in yet another aspect, a method of manufacturing a biosensor for *use in vivo* to monitor changes along the interface between living tissue and an implant.

In yet another aspect, the invention provides for a method for integrating a biosensor with a medical implant for use in monitoring adjacent tissue changes and identifying said tissue following implantation.

Disclosed below is the further novel evaluation undertaken by the inventors that more fully describes the embodiments of the present invention of a method for treating a surface of a titanium medical implant that results in the formation of integral biosensors. In addition, the evaluation discloses a medical implant that includes integral biosensors that are capable of sensing the conductivity of surrounding tissue, including bone and other types of tissue.

In order to form a more robust interconnection, as has been described above, the inventors have anchored CNTs in the pores of anodized nanotubular Ti in this evaluation. Then, multi-walled carbon nanotubes (hereinafter "MWCNTs") were grown, using CVD techniques, out of anodized Ti nanotubes (with diameters of 50-60 nm and depths of 200 nm) as a template. In electronic theory, when two different materials come in contact with each other, electron transfer will occur in an attempt to balance Fermi levels, causing the formation of a double layer of electrical charge at the interface. Herein, electron transfer between Ti-based electrodes and electrolyte solutions, which contained Calcium (Ca) and Iron (Fe) ions were mainly observed. However, the electron transfer between the interface of Ti-based electrodes and osteoblast cells in tissue culture, was also examined in this evaluation.

## *MATERIALS AND METHODS*

### 1. Ti-based Electrodes

Commercially pure Ti (Ti $_{micro}$; Alfa Aesar), anodized Ti, and MWCNT-Ti were used as a working electrode with a geometric area of 1 mm$^2$ and 1 cm$^2$. The anodized Ti (Ti$_{nano}$) was prepared by anodization techniques. Briefly, 1 cm$^2$ of Ti was etched with a solution of 1.5% by weight nitric acid and 0.5% by weight hydrofluoric acid (HF) for ten (10) seconds. A potential of twenty (20) volts was applied between a Ti and platinum (Pt; Alfa Aesar) sheet electrode for ten (10) minutes in the presence of 1.5 % by weight HF in order to create uniform nanopores. Afterward Ti$_{nano}$ were further modified by growing MWCNTs out of the nanopores via CVD. Deionized water was used to clean all electrodes before the electrochemical measurements.

### 2. Osteoblast Culture and Calcium-Contained Electrolyte Solutions

Human osteoblasts (CRL-11372; ATCC; population number = 5-12) were cultured in Dulbecco's Modified Eagle Medium (DMEM; Gibco) supplemented with 10% fetal bovine serum (FBS; Hyclone), 1% penicillin/streptomycin (P/S; Hyclone), 50 nM β-glycerophosphate (Sigma), and 50 μg/ml ascorbic acid (Sigma) for seven (7), fourteen (14), and twenty-one (21) days on commercially pure Ti with an initial cell density of 40,000 cells/cm$^2$ under standard cell culture conditions (a humidified, 5% $CO_2$, and 95% air environment at 37° C). After each time period and three freeze-thaw cycles with distilled water to lyse the cells, the calcium-containing electrolyte solution was prepared by incubating Ti substrates after each culture period with 0.6 N HCl (Sigma) for 24 hours. A calcium quantification kit (Sigma) was used to determine the amount of calcium deposited by osteoblasts. The light absorbance of calcium in the supernatants was measured by a spectrophotometer (SpectraMAX 340PC$^{384}$; Molecular Devices) at 570 nm. The chemical composition on the surfaces of the Ti substrates after osteoblast culture for twenty-one (21) days was also evaluated by energy dispersive spectroscopy (SEM-EDS; Leo 1530VP FE-4800). Osteoblasts were cultured separately on another set of Ti and anodized Ti substrates for fourteen (14) days in order to test the electrochemical behavior of Ti$_{micro}$/Ti$_{nano}$ substrates.

### 3. Electrochemical Measurements

The cyclic voltammetry experiments were performed by using an Epsilon EC potentiostat and Digisim software (Bioanalytical System). Both MWCNT-Ti and Ti were used as the working electrodes in this study. The working electrode area was 1 mm$^2$ and 1 cm$^2$ which were demarcated by Teflon tape. A silver/silver chloride (Ag/AgCl; MW-2052; Bioanalytical System) and platinum (MW-1032; Bioanalytical System) wire were used as a reference and counter electrodes, respectively. Before the measurements, all electrodes were cleaned with deionized water. Three types of the electrodes were connected to the potentiostat and immersed in an electrolyte solution. Two kinds of electrolyte solutions were used in order to analyze and compare the electrolyzed oxidation states of MWCNT-Ti electrodes. The first aqueous electrolyte was a 10 mM $K_3Fe(CN)_6$ (potassium ferricyanide) solution with 1 M $KNO_2$. To obtain real calcium deposited from osteoblasts, not only were calcium-contained supernatants used for calcium determination by light absorbance, but they were also used as the second electrolyte solution, which was calcium dissolved in 0.6 N HCl. Simply, conventional Ti (Ti$_{micro}$) was used as the substrates for cell culture, and then calcium deposited by osteoblasts was collected at the end of seven (7), fourteen (14), and twenty-one (21) days.

The cyclic voltammograms (hereinafter "CV") were generated between -1000 mV to 1000 mV by applying a linear sweep potential at several scan rates. The CV second cycle was recorded to obtain the steady-state CVs, as well as the capacitance and charge-transfer capacitance of the electrodes and electrolyte solutions. All measurements were carried out at room temperature.

## *RESULTS AND DISCUSSIONS*

### 1. Electrode Topography

The conventional Ti (Ti$_{micro}$) surface as shown in FIG. 7(a) exhibited a smooth Ti oxide at the nanoscale. After anodization, the nanopores of Ti oxide were formed on the Ti surface (Ti$_{nano}$) uniformly, as shown in FIG.7(b), with diameters of 50-60 nm and depths of 200 nm. After CVD, MWCNTs covered the Ti$_{nano}$ templates as shown in FIG. 7(c) side view and (d) top view.

### 2. Calcium Analysis

Results from energy dispersive spectroscopy (hereinafter "EDS") performed on commercially pure Ti substrates after osteoblasts were cultured for twenty-one (21) days revealed the presence of various minerals in newly formed bone, specifically magnesium (Mg), phosphorus (P), sulphur (S), potassium (K), and calcium (Ca) (See FIG. 8). The Ca/P weight ratio of mineral deposited by osteoblasts on commercial Ti (1.34) was less than that on MWCNT-Ti (1.52). The Ca/P ratio of hydroxyapatite (HA), the main calcium-phosphate crystallite in bone, is about 1.67. The evaluation demonstrated that mineral deposited by osteoblasts on MWCNT-Ti was more similar to natural bone than mineral deposited on Ti.

X-ray diffraction (hereinafter "XRD") analysis of conventional Ti and MWCNT-Ti after osteoblasts were cultured for twenty-one (21) days, showed hydroxyapatite deposited on MWCNT-Ti more than conventional Ti and anodized Ti. In addition, the amount of calcium deposited by osteoblasts as determined by a calcium quantification assay kit was 1.481 $\mu g/cm^2$ after 7 days, 1.597 $\mu g/cm^2$ after fourteen (14)days, and 2.483 $\mu g/cm^2$ after twenty-one (21) days on conventional Ti. Importantly, these results indicated a greater deposition of calcium by osteoblasts on MWCNT-Ti.

### 3. Electrochemical Behavior of $Fe^{2+/3+}$ Redox Couple at the $Ti_{micro}/Ti_{nano}$ and MWCNT-Ti Electrodes

The $Fe(CN)_6^{4-/3-}$ redox system is one of the most extensively studied redox couples in electrochemistry. In this evaluation, the CVs of $Fe(CN)_6^{4-/3-}$ couples were electrolyzed by placing in a solution of 10 mM $K_3Fe(CN)_6$ and 1 M $KNO_3$ in contact with Ti-based electrode surfaces, as shown in FIG. 10. In potassium ferricyanide ($K_3Fe(CN)_6$), the reduction process is $Fe^{3+}$ ($Fe(CN)_6^{3-} + e^- \rightarrow Fe(CN)_6^{4-}$) followed by the oxidation of ($Fe(CN)_6^{4-} \rightarrow Fe(CN)_6^{3-} + e^-$) under a sweeping voltage. The iron(II/III) redox couple does not exhibit any observable peaks for bare $Ti_{micro}$ and $Ti_{nano}$ electrodes, as shown in FIGS. 10(a) and 10(b), indicating that its adsorption on $Ti_{micro}$ and $Ti_{nano}$ surfaces was weak and the electrochemical reaction was slow on both electrodes as a result of fewer electron transfer through the electrode surface. However, the pair of redox peaks was observed, as shown in FIG. 10(c) when using MWCNT-Ti as a working electrode. A well defined redox peak appears at a scan rate of 100 mV/s. For example, the anodic ($E_{pa}$) and cathodic ($E_{pc}$) peak potentials of MWCNT-Ti appear at 175 mV and 345 mV as shown in FIG. 10(c). On the inner set of FIG. 10(c), the relationship between anodic and cathodic peak currents versus scan rate square root is linear with the ratio of $I_{pa}/I_{pc}$ at about 1, indicating that the MWCNT-Ti electrode is quasi-reversible ($\Delta E_p > 59/n$ mV; n = one electron transferred in $Fe^{2+/3+}$ redox couple process) under this condition.

From these results is can be concluded that MWCNTs have good electrochemical characteristics as electron mediators and adsorption matrices [31], thus, possibly enhancing applications in biosensor systems. As note previously, other studies have indicated the possible use of CNT-modified electrodes for biosensing purposes. It has been shown that using CNTs may be a promising method to enhance detection sensitivity because they have high signal-to-noise ratios. The structure-dependent metallic character of CNTs should allow them to promote electron-transfer reactions for redox reactions, which can provide the foundation for unique biochemical sensing systems at low over-potentials. The electrolyte-electrode interface barriers have been reduced by CNTs, because they facilitate double-layer-effects. Typically, when the supporting electrolyte is in excess, at least a hundred-fold greater than the active electrolyte, the charge in the electrolyte solution causes the Debye layer to be more compact and rapidly exchange electrons with electroactive species, leading to sharpened cathodic and anodic peaks to be observed in CV.

Evaluating the electron transfer from MWCNTs grown out of Ti, herein, the electroactive species Ca and Fe ions were used. Moreover, the MWCNT-Ti electrode exhibited a high electroactive surface area according to the Randles-Sevcik equation for quasi-reversible and reversible process showed below, mainly due to the presence of MWCNTs that acted as a nanobarrier between the $TiO_{2nano}$ surface, which was the MWCNT growth template, and the electrolyte solution. For the quasi-reversible system (with $10^{-1} > k > 10^{-5}$ cm/s), the current was controlled by both the charge transfer and mass transport. The shape of the CV for this quasi-reversible system of MWCNT-Ti was more extended, as shown in FIG. 10(c), and exhibited a large separation in peak potentials; $\Delta E_p > 59$ mV. The peak current ($I_p$) is given by:

$$I_p = 2.99 \times 10^5 \, AD^{1/2} n(n_a \gamma)^{1/2} C \qquad (1)$$

Where $n$ is the number of electrons participating in redox process, $n_a$ is the number of electrons participating in the charge-transfer step, $A$ is the area of the working electrode ($cm^2$), D is the diffusion coefficient of the molecules in the electrolyte solution ($cm^2/s$), C is the concentration of the probe molecule in the bulk solution (molar), and y is the scan rate of the sweep potential (V/s). When the $Fe(CN)_6^{4-/3-}$ redox system exhibits a heterogeneous one-electro transfer ($n$=1) and the concentration (C) is equal to 10 mM, the diffusion coefficient (D) is equal to 6.70 $\pm$ 0.02 x $10^{-6}$ $cm^2$/s.

As depicted in FIG. 10(a), a weak CV signal from the $Ti_{micro}$ electrode showed the possible anodic peak near 0 V, and the $Ti_{nano}$ electrode did not show any peaks. The increased surface area (A) by the formation of nanopores on Ti ($Ti_{nano}$), might increase the double layer effect between the electrode and electrolyte solution, leading to an increase in the impedance of the electrode. As exhibited in FIG. 10(d), the capacitance of the $Ti_{micro}$ electrode in this system is more than the $Ti_{nano}$ electrode. The $Ti_{micro/nano}$ electrode does not display obvious redox curves, likely due to inert properties of $TiO_2$ in the biological medium at various pH levels. FIGS. 10(a), (b), and (c) showed that $Ti_{nano}$ has less capacitance than $Ti_{micro}$. Hence, $Ti_{nano}$ might cause more double layer effects on its surface in the electrolyte solutions, leading to a decrease in the number of electrons passing through the electrode surfaces.

**4. Electrochemical Behavior of Calcium Ion Redox Couple at the $Ti_{micro}$/$Ti_{nano}$ and MWCNT-Ti Electrodes**

This evaluation showed that calcium deposited by osteoblasts was detected only when using MWCNT-Ti as a working electrode, as evidenced in FIGS. 11 and 12. For calcium ions, CV also confirmed that there was no detection of calcium when using $Ti_{micro/nano}$, as shown in FIGS. 11(a) and 11(b). The effect of the working electrode area was also investigated. Regarding the Randles-Sevcik equation, the peak current was directly proportional to the area. The current in FIG. 11 was 10 times greater and also the area was ten (10) times greater than that in FIG. 12. When plotting the anodic ($I_{pa}$) and cathodic peak ($I_{pc}$) current of MWCNT-Ti, a linear relationship with scan rates was observed, as shown in FIG. 12(c). The capacitance of MWCNT-Ti and Ti were calculated by divided with respect to specific scan rate, as compared in FIG. 12(d). The result from the iron(II/III) redox couple showed the potential in using MWCNT-Ti as an electrode, thus, the different concentration of calcium deposited by osteoblasts for seven (7), fourteen (14), and twenty-one (21) days, was also investigated. In FIGS. 11(c) and 12(a), CV showed the quasi-reversible redox potential in the calcium-containing solution of concentration of 2.48 $\mu g/cm^2$ after osteoblasts were cultured for twenty-one (21) days. While the calcium concentration after seven (7) days of mineral deposition was 1.481 $\mu g/cm^2$ and fourteen (14) days was 1.597 $\mu g/cm^2$, both concentrations did not show any possible redox potential peaks. However, their shapes were still more extended and proportional to the bulk concentration, as shown in FIGS. 13(a) and 13(b), corresponding to the Randles-Sevcik equation.

**5. Electrochemical Behavior of Osteoblasts Media Ions at the $Ti_{micro}$/$Ti_{nano}$ Electrodes**

After osteoblasts were cultured on $Ti_{micro}$ and $Ti_{nano}$, these substrates were used to generate CV as shown in FIGS. 13(a) and (b). Transmembrane potentials of osteoblasts *in vivo* have been studied to understand the ionic movements through osteoblast membranes. For example, Jeansonne et al. found that these membrane potential responses indicate the change in $Ca^{2+}$ handling by osteoblasts. Osteoblasts exhibited a uniquely low polarization of their cell membranes (-3.93 mV) and indicated transient changes in osteoblast membrane potentials. In FIG. 13(a)), CV showed an irreversible process because there was only the anodic peak, which was around -5 to -4 mV depending on the scan rate. The transient changes of current and potential in CV also confirmed those in the Jeansonne et al. study. The lower the scan rate, the more obvious the anodic peaks were. Hence, the low potential can promote calcium ion transfer through osteoblast membranes, leading to induced calcium deposition with low voltage. It was found during the evaluation that osteoblasts cultured on $Ti_{micro/nano}$ and MWCNT-Ti died after applying a voltage of 25-50 mV/s for twenty (20) minutes a day. It can be hypothesized that a very low potential (< 25 mV/s) is more suitable for osteoblast viability. In another words, electrical stimulation with low voltage (<100 mV/s) induced electrons to pass through osteoblast membranes, enhancing the electron transfer, or the calcium ion redox couple.

**6. Applications and Alternative Embodiments**

Surface modification and growing MWCNTs from metals can lead to more versatile applications. For example, MWCNTs functionalized with thiol groups have been used for sensing aliphatic hydrocarbons (such as methanol, ethanol, proponal and butanol) forming unique electrical identifiers. MWCNTs grown on silicon substrates and integrated with unmodified plant cellulose as a film in both a room temperature ionic liquids (RTIL) and bioelectrolytes, such as body sweat and blood were used as a supercapacitor in biological fluid at wide working temperatures of 195-423 K, which was better than any commercially available supercapacitor (233-358 K) due to their enhanced ionic conductivity. Therefore, the invention may also work here if used as a dry-body implant with much wider operating temperature ranges (such as to determine in vitro calcium adsorption). That is for example only, after a hip implant insertion, blood and body fluids surround the Ti implant, creating a specific capacitance for commercially pure Ti. Within one month, osteoblasts will deposit calcium and the capacitance at the interface of the medical implant and bone tissue will increase because the deposited calcium may surround the medical implant. From the present in vitro evaluation results, the capacitance of Ti and anodized Ti electrodes increased in the ionic osteoblast media after being cultured for fourteen (14) days, leading to the possibility of enhanced capacitance after bone tissue is formed *in vivo.*

Moreover, this film without electrolytes can serve as a cathode electrode in a lithium ion battery. Interestingly, the supercapacitor and battery, derived from a nanocomposite film, can be integrated together to build a hybrid, or dual-storage battery-in-supercapacitor device. The discharge of the battery is used to charge the supercapacitor because the ion double layer is formed at the surface of the battery cathode, and then forms the electrical double layer, which is discharged later in the supercapacitor mode, formed at the supercapacitor electrode.

In addition, any implanted electronics may be powered by the induction of an external power supply at low frequency pulse, or by implant battery (fabricated or self-integrated within the implant material). The low frequency of the inductive power source facilitates power transmission through the metal medical implant. A telemetry system *in vivo* with a small implantable transmitter could also use a high frequency pulse (such as radio frequency; RF) in order to transmit a signal to an external device. The sensor signals might have to be multiplexed and modulated at a specific RF, before being transmitted to an external device. To transmit the pulse interval modulated signal, a pacemaker feed-through forms a single loop antenna outside the metal case at one end of the implant. A microcontroller in an external device alternates a magnetic field, produced *in vivo,* with a power oscillator. It synchronizes the modulated pulse interval to recover the data stream. A system programming sensor microcontroller is also important to control its working capabilities. For example, for orthopedic applications, Friedmar et al. developed a new 9-channel telemetry transmitter used for *in vivo* load measurements in three patients with shoulder Ti endoprostheses. Telemetric devices for orthopedic application started in 1966. The telemetry and their applicable potentials are imperative for orthopedic application, which can be applied for developing a chip for calcium measurements for clinic use in the future.

### *CONCLUSIONS*

The capacitance of Ti in an aqueous system increased in the evaluation disclosed herein by anodization and growing MWCNTs out of anodized Ti nanopores. MWCNTs extended the redox potential when compared to bare Ti and anodized Ti. These results provide evidence that MWCNTs can be used as a novel electrode through their growth out of nanoporous anodized Ti due to their increasing capacitance. CV confirmed the redox peaks on MWCNT-Ti, likely due to the fact that MWCNTs improved electron transfer through the electrode when compared with bare Ti (both conventional and anodized). They enhanced the redox potential by enhancing the electron transfer in ionic solutions in the presence of the electroactive species, such as ferri/ferricyanide and calcium (deposited by osteoblasts). A previous study found that MWCNTs are cytocompatible and promoted osteoblast differentiation after twenty-one (21) days. Also, it is possible, that MWCNTs may be integrated into a supercapacitor or battery, enhancing the device's conductivity *in vivo.* Therefore, MWCNT-Ti can be considered as an electrode to determine new bone growth *in situ* surrounding an orthopedic implant and may be used in other medical applications to detect the presence and type of other tissues including but not limited to, soft, connective, including collagen, tendon, cartilage and other biological precursors of these tissue types. The ability of electrodes to sense calcium (deposited by osteoblasts) in specific concentrations might improve the diagnosis of orthopedic implant success or failure and, thus, improve clinical efficacy.

Various patent and/or scientific literature references have been referred to throughout the instant specification. In view of the detailed description of the invention, one of ordinary skill in the art will be able to practice the invention as claimed without undue experimentation. Other aspects, advantages, and modifications are within the scope of the following claims as will be apparent to those skilled in the art.

### *References*

Ager III, J. W.; Balooch, G.; and R.O., 2006 Fracture, aging, and disease in bone, Journal of Materials Research 21, 1878-92.

Ward, B. C.; and Webster, T. J., 2006 The effect of nanotopography on calcium and phosphorus deposition on metallic materials, in vitro Biomaterials 27, 3064-74.

Liu, H.; and Webster, T. J., 2007 Nanomedicine for implants: A review of studies and necessary experimental tools, Biomaterials 28, 354-369.

Orosz, GM.; Hannan, E.; Magaziner, J.; Koval, K.; Gilbert, M.; Aufses, A.; Straus, E.; Vespe, E.; and Siu, A. L., 2002 Hip fracture in the older patient: Reasons for delay in hospitalization and timing of surgical repair, Journal of the American Geriatrics Society 50, 1336-1340.

Yao, C.; Perla, V.; McKenzie, J. L.; Slamovich, E. B.; and Webster, T. J., 2005 Anodized Ti and Ti6A1V possessing nanometer surface features enhance osteoblast adhesion, Journal of Biomedical Nanotechnology 1, 68-73.

Zhu, X.; Chen, J.; Scheideler, L.; Reichl, R.; and Geis-Gerstorfer, J., 2004 Effects of topography and composition of titanium surface oxides on osteoblast responses, Biomaterials 25, 4087-103.

Bekyarova, E.; Ni, Y.; Malarikey, E. B.; Montana, V.; McWilliams, J. L.; Haddon, R. C.; and Parpura, V., 2005 Applications of carbon nanotubes in biotechnology and biomedicine, Journal of Biomedical Nanotechnology 1, 3-17.

Khang, D.; Sato, M.; Price, R. L.; Ribbe, A. E.; and Webster, T. J., 2006 Selective adhesion and mineral deposition by osteoblasts on carbon nanofiber patterns, International Journal ofNanomedicine 1, 65-72.

Zanello, L.P.; Zhao, B.; Hu, H.; and Haddon, R.C., 2006 Bone cell proliferation on carbon nanotubes, Nano Letters 1, 19-22.

Zanello, L.P., 2006 Electrical properties of osteoblasts cultured on carbon nanotubes, Micro & Nano Letters 1, 19-22.

Tsuchiya, N.; Sato, Y.; Aoki, N.; Yokoyama, A.; Watari, F.; Motomiya, K.; Jeyadevan, B.; and Tohji, K., 2007 Evaluation of multi-walled carbon nanotube scaffolds for osteoblast-like cells, AIP Conference Proceeding 898, 166-169.

Ciombor, D. M. and Aaron, R. K., 1993 Influence of electromagnetic fields on endochondral bone formation, Journal of Cell Biochemistry 52, 37-41.

Ciombor, D. M. and Aaron, R. K., 2005, The role of electrical stimulation in bone, repair Foot Ankle Clinical 10, 579-93.

Christenson, E. M.; Anseth, K. S.; van den Beucken, J. J. J. P.; Chan, C. K.; Ercan, B.; Jansen, J. A.; Laurencin, C. T.; Li, W. J.; Murugan, R.; Nair, L. S.; Ramakrishna, S.; Tuan, R. S.; Webster, T. J.; and Mikos, A. G., 2006 Nanobiomaterial applications in orthopedics Journal of Orthopedic Research 10, 1-12.

Webster, T. J., 2004 Increased osteoblast adhesion on nanophase metals: Ti, Ti6A14V, and CoCrMo, Biomaterials 25, 4731-39.

Zhao, B.; Hu, H.; Mandal, S. K., Haddon, R. C.; 2005 A bone mimic based on the self-assembly of hydroxyapatite on chemically functionalized single walled carbon nanotubes Chemistry of Materials 17 3235-41

Supronowicz, P. R.; Ajayan, P. M.; Ullmann, K. R.; Arulanandam, B. P.; Metzger, D. W.; Bizios, R., 2002 Novel current-conducting composite substrates for exposing osteoblasts to alternating current stimulation, Journal of Biomedical Materials Research 59, 499-506.

Besteman, K.; Lee, J. O.; Wiertz, F, G.; Heering, H. A.; Dekker, C., 2003 Enzyme coated carbon nanotubes as single-molecule biosensors, Nano Letters 3, 727-30.

Harrison, B. S.; and Atala, A., 2007 Carbon nanotube applications for tissue engineering, Biomaterial 28, 344-53.

Rochefort, A.; Avouris, P.; Lesage, F.; Salahub, D. R., 1999 Electrical and mechanical properties of distorted carbon nanotubes, Physical Review B 60, 824-30.

Kreupl, F.; Graham, A. P.; Duesberg, G. S.; Steinhögl, W.; Liebau, M.; Unger, E.; and Hönlein, W., 2002 Carbon nanotubes in interconnect applications, Microelectronic Engineering 64, 399-408.

Zhan, C.; Kaczmarek, R.; Loyo-Berrios, N.; Sangl, J.; Bright, R. A., J. Bone Joint Surg. Am. 2007, 3, 526-533.

Iijima, S. Nature 1991, 6348, 56-58.

Lin, Y.; Taylor, S.; Li, H.; Fernando, K. A. S.; Qu, L.; Wang, W.; Gu, L.; Zhou, B.; Sun, Y., J. Mater. Chem. Y1 - 2004///, 4, 527-541.

Webster, T. J.; Waid, M. C.; McKenzie, J. L.; Price, R. L.; Ejiofor, J. U., Nanotechnology 2004, 1, 48-54.

Smart, S. K.; Cassady, A. I.; Lu, G. Q.; Martin, D. J., Carbon 2006/5, 6, 1034-1047.

[00100] Zanello, L. P.; Zhao, B.; Hu, H.; Haddon, R. C., Nano Lett. 2006, 3, 562-567.

Zanello, L. P. Micro Nano Lett. 2006, 1, 19-22.

Balani, K.; Anderson, R.; Laha, T.; Andara, M.; Tercero, J.; Crumpler, E.; Agarwal, A., Biomaterials 2007/2, 4, 618-624.

Chen, Y.; Zhang, T. H.; Gan, C. H.; Yu, G., Carbon 2007/4, 5, 998-1004.

Wei, W.; Sethuraman, A.; Jin, C.; Monteiro-Riviere, N. A.; Narayan, R. J., Journal of Nanoscience and Nanotechnology April/May 2007, 1284-1297(14).

Harrison, B. S.; Atala, A., Biomaterials 2007/1, 2, 344-353.

Supronowicz, P. R.; Ajayan, P. M.; Ullmann, K. R.; Arulanandam, B. P.; Metzger, D. W.; Bizios, R., J. Biomed. Mater. Res. 2002, 3, 499-506.

Ciombor, D. M.; Aaron, R. K., Foot and Ankle Clinics of North America 2005/12, 4, 579-593.

Aoki, N.; Yokoyama, A.; Nodasaka, Y.; Akasaka, T.; Uo, M.; Sato, Y.; Tohji, K.; Watari, F., Journal of Biomedical Nanotechnology December 2005, 402-405(4).

Giannona, S.; Firkowska, I.; Rojas-Chapana, J.; Giersig, M., Journal of Nanoscience and Nanotechnology April/May 2007, 1679-1683(5).

Tsuchiya, N.; Sato, Y.; Aoki, N.; Yokoyama, A.; Watari, F.; Motomiya, K.; Jeyadevan, B.; Tohji, K., In Evaluation of Multi-Walled Carbon Nanotube Scaffolds for Osteoblast Growth; Tohji, K., Tsuchiya, N. and Jeyadevan, B., Eds.; AIP: 2007; Vol. 898, pp 166-169.

Sirivisoot, S.; Yao, C.; Xiao, X.; Sheldon, B. W.; Webster, T. J., Nanotechnology 2007, 36, 365102.

Padigi, S. K.; Reddy, R. K. K.; Prasad, S., Biosensors and Bioelectronics 2007/1/15, 6, 829-837.

Roy, S.; Vedala, H.; Choi, W., Nanotechnology 2006, 4, S14-S18.

Tang, H.; Chen, J. H.; Huang, Z. P.; Wang, D. Z.; Ren, Z. F.; Nie, L. H.; Kuang, Y. F.; Yao, S. Z., Carbon 2004, 1, 191-197.

Kurusu, F.; Koide, S.; Karube, I.; Gotoh, M., Anal. Lett. 2006, 903-911.

Liu, Y.; Wang, M.; Zhao, F.; Xu, Z.; Dong, S., Biosensors and Bioelectronics 2005/12/15, 6, 984-988.

Liu, S.; Lin, B.; Yang, X.; Zhang, Q. J., Phys. Chem. B 2007, 5, 1182-1188.

Robertson, J., Material Today 2004/10, 10, 46-52.

Talapatra, S Kar, S.; Pal, S. K.; Vajtai J.; Ci S.; Victor, P.; Shaijumon, M. M.; Kaur, S.; Nalamasu, O.; Ajayan, P. M., Nat Nano, 112-116.

Ngo, Q.; Petranovic, D.; Krishnan, S.; Cassell, A. M.; Ye, Q.; Li, J.; Meyyappan, M.; Yang, C. Y., Nanotechnology, IEEE Transactions on 2004, 311-317.

Sato, S.; Kawabata, A.; Kondo, D.; Nihei, M.; Awano, Y., Chemical Physics Letters 2005/1/24, 1-3, 149-154.

Mathiowitz, E., Encyclopedia of Controlled Drug Delivery; John Wiley & Sons: 1999; Vol. 1-2, pp 1120.

Calafiori, A.; Di Marco, G.; Martino, G.; Marotta, M., J. Mater. Sci. Mater. Med.

Wang, H.; Lee, J.; Moursi, A.; Lannutti, J. J., Journal of Biomedical Materials Research Part A 2003, 2, 599-608.

Sotiropoulou, S.; Gavalas, V.; Vamvakaki, V.; Chaniotakis, N. A., Biosensors and Bioelectronics 2003/3, 2-3, 211-215.

Fang, W. C.; Sun, C. L.; Huang, J. H.; Chen, L. C.; Chyan, O.; Chen, K. H.; Papakonstantinou, P., Electrochem. Solid State Lett. 2006, 3, A175-A178.

G.D. Christian, Analytical Chemistry; John Wiley & Sons: New York, 1980;.

Allen J. Bard; Larry R. Faulkner, Electrochemical Methods: Fundamentals and Applications; Wiley: 2000; , pp 856.

Hrapovic, S.; Liu, Y.; Male, K. B.; Luong, J. H. T., Anal. Chem. 2004, 4, 1083-1088.

Hrapovic, S.; Luong, J. H. T., Anal. Chem. 2003, 14, 3308-3315.

Jeansonne, B. G.; Feagin, F. F.; McMinn, R. W.; Shoemaker, R. L.; Rehm, W. S., J. Dent. Res. 1979, 4, 1415-1423.

Pushparaj, V.; Shaijumon, M.; Kumar, A.; Murugesan, S.; Ci, L.; Vajtai, R.; Linhardt, R.; Nalamasu, O.; Ajayan, P., Proceedings of the National Academy of Sciences 2007, 34, 13574-13577.

Graichen, F.; Arnold, R.; Rohlmann, A.; Bergmann, G., IEEE Trans. Biomed. Eng. 2007, 2, 253-261.

Kaufman, K.; Kovacevic, N.; Irby, S.; Colwell, C., Journal of Biomechanics 1996, 5, 667-671.

D'Lima, D.; Townsend, C.; Arms, S.; Morris, B.; Colwell, C., Journal of Biomechanics 2005, 2, 299-304.

Bergmann, G.; Deuretzbacher, G.; Heller, M.; Graichen, F.; Rohlmann, A.; Strauss, J.; Duda, G. N., Journal of Biomechanics 2001, 7, 859-871.

Bergmann, G.; Graichen, F.; Rohlmann, A.; Verdonschot, N.; van Lenthe, G. H., J. Biomech. 2001, 4, 421-428.

Graichen, F.; Bergmann, G.; Rohlmann, A., Journal of Biomechanics 1999, 10, 1113-1117.

Graichen, F.; Bergmann, G., Journal of Biomedical Engineering 1991, 5, 370-374.

## Claims

1. A medical implant having enhanced cytocompatibility capabilities, the medical implant comprising:

    a titanium metallic substrate having an outer surface and wherein said outer surface comprises a composition of nanosized structures attached thereto, said nanosized structures being a plurality of nanotubes that are integrally attached to said metallic substrate, the integrally attached nanotubes each having a lumen and further having multi-walled carbon nanotubes growing within said lumens, whereby the multi-walled carbon nanotubes cause the medical device to have enhanced cytocompatibility capabilities.

2. A medical implant according to claim 1 having a biosensor integrated therewith, the biosensor comprising an electrode configured to be integrally coupled to an outer surface of the medical implant, wherein the biosensor detects electrochemical changes adjacent to the medical implant.

3. A medical implant according to claim 2, wherein the biosensor electrode comprises at least one nanostructure.

4. A medical implant according to claim 3, wherein the at least one nanostructure of the biosensor further comprises at least one multi-walled carbon nanotube and at least one nanotube, wherein the at least one multi-walled carbon nanotube is positioned inside of the at least one nanotube.

5. A medical implant according to claim 2, wherein the biosensor detects the conductivity of tissue positioned adjacent to the medical implant.

6. A medical implant according to claim 5, wherein the level of conductivity detected by the biosensor identifies:

   (a) the tissue type positioned adjacent to the medical implant, and/or;
   (b) the presence of tissue positioned adjacent to the medical implant.

7. A method of manufacturing a medical implant with a biosensor, the method comprising:

   obtaining a medical implant; and
   treating a surface of the medical implant to modify the surface characteristics resulting in the formation of a biosensor attached to the surface of the medical implant; wherein the treating a surface of the medical implant further comprises anodizing the surface and performing a chemical vapor deposition process, wherein the treating a surface results in growing a plurality of multi-walled carbon nanotubes within the lumens of a plurality of anodized nanotubular structures.

8. The method of claim 7, wherein the plurality of multi- walled carbon nanotubes in combination within the plurality of anodized nanotubular structures comprises the biosensor that is attached to the surface of the medical implant.


**Patentansprüche**

1. Ein medizinisches Implantat mit verbessertem Zytokompatibilitätsvermögen, wobei das medizinische Implantat Folgendes beinhaltet:

   ein Titanmetallsubstrat mit einer äußeren Oberfläche und wobei die äußere Oberfläche eine Zusammensetzung aus daran angebrachten nanogroßen Strukturen beinhaltet,
   wobei die nanogroßen Strukturen eine Vielzahl von Nanoröhrchen sind, die an dem Metallsubstrat integral angebracht sind, wobei die integral angebrachten Nanoröhrchen jeweils ein Lumen aufweisen und ferner mehrwandige Kohlenstoffnanoröhrchen aufweisen, die innerhalb der Lumina wachsen, wobei die mehrwandigen Kohlenstoffnanoröhrchen bewirken, dass die medizinische Vorrichtung ein verbessertes Zytokompatibilitätsvermögen aufweist.

2. Medizinisches Implantat gemäß Anspruch 1 mit einem darin integrierten Biosensor, wobei der Biosensor eine Elektrode beinhaltet, die konfiguriert ist, um mit einer äußeren Oberfläche des medizinischen Implantats integral gekoppelt zu sein, wobei der Biosensor elektrochemische Änderungen neben dem medizinischen Implantat detektiert.

3. Medizinisches Implantat gemäß Anspruch 2, wobei die Biosensorelektrode mindestens eine Nanostruktur beinhaltet.

4. Medizinisches Implantat gemäß Anspruch 3, wobei die mindestens eine Nanostruktur des Biosensors ferner mindestens ein mehrwandiges Kohlenstoffnanoröhrchen und mindestens ein Nanoröhrchen beinhaltet, wobei das mindestens eine mehrwandige Kohlenstoffnanoröhrchen innerhalb des mindestens einen Nanoröhrchens positioniert ist.

5. Medizinisches Implantat gemäß Anspruch 2, wobei der Biosensor die Leitfähigkeit von neben dem medizinischen Implantat positionierten Gewebe detektiert.

6. Medizinisches Implantat gemäß Anspruch 5, wobei der von dem Biosensor detektierte Grad an Leitfähigkeit Folgendes identifiziert:

   (a) den neben dem medizinischen Implantat positionierten Gewebetyp und/oder;
   (b) das Vorhandensein von neben dem medizinischen Implantat positionierten Gewebe.

7. Ein Verfahren zum Herstellen eines medizinischen Implantats mit einem Biosensor, wobei das Verfahren Folgendes beinhaltet:

Erhalten eines medizinischen Implantats; und

Behandeln einer Oberfläche des medizinischen Implantats, um die Oberflächeneigenschaften zu ändern, was die Bildung eines an der Oberfläche des medizinischen Implantats angebrachten Biosensors zur Folge hat; wobei das Behandeln einer Oberfläche des medizinischen Implantats ferner das Anodisieren der Oberfläche und das Durchführen eines Vorgangs der chemischen Gasphasenabscheidung beinhaltet, wobei das Behandeln einer Oberfläche das Wachsen einer Vielzahl von mehrwandigen Kohlenstoffnanoröhrchen innerhalb der Lumina einer Vielzahl von anodisierten Nanoröhrenstrukturen zur Folge hat.

8. Verfahren gemäß Anspruch 7, wobei die Vielzahl von mehrwandigen Kohlenstoffnanoröhrchen in Kombination innerhalb der Vielzahl von anodisierten Nanoröhrenstrukturen den Biosensor beinhaltet, der an der Oberfläche des medizinischen Implantats angebracht ist.

## Revendications

1. Un implant médical ayant des capacités de cytocompatibilité accrues, l'implant médical comprenant :

un substrat métallique en titane ayant une surface externe et dans lequel ladite surface externe comprend une composition de structures nanométriques attachées à celle-ci, lesdites structures nanométriques étant une pluralité de nanotubes qui sont attachés de façon solidaire audit substrat métallique, les nanotubes attachés de façon solidaire ayant chacun une lumière et ayant en sus des nanotubes de carbone multiparois qui croissent au sein desdites lumières, les nanotubes de carbone multiparois donnant de ce fait au dispositif médical des capacités de cytocompatibilité accrues.

2. Un implant médical selon la revendication 1 auquel est intégré un biocapteur, le biocapteur comprenant une électrode configurée pour être couplée de façon solidaire à une surface externe de l'implant médical, dans lequel le biocapteur détecte des changements électrochimiques au voisinage de l'implant médical.

3. Un implant médical selon la revendication 2, dans lequel l'électrode du biocapteur comprend au moins une nanostructure.

4. Un implant médical selon la revendication 3, dans lequel cette au moins une nanostructure du biocapteur comprend en sus au moins un nanotube de carbone multiparoi et au moins un nanotube, dans lequel cet au moins un nanotube de carbone multiparoi se trouve à l'intérieur de cet au moins un nanotube.

5. Un implant médical selon la revendication 2, dans lequel le biocapteur détecte la conductibilité de tissu se trouvant au voisinage de l'implant médical.

6. Un implant médical selon la revendication 5, dans lequel le niveau de conductibilité détecté par le biocapteur identifie :

(a) le type de tissu se trouvant au voisinage de l'implant médical, et/ou ;
(b) la présence de tissu se trouvant au voisinage de l'implant médical.

7. Un procédé de fabrication d'un implant médical avec un biocapteur, le procédé comprenant :

l'obtention d'un implant médical ; et
le traitement d'une surface de l'implant médical afin de modifier les caractéristiques de surface entraînant la formation d'un biocapteur attaché à la surface de l'implant médical ; dans lequel le traitement d'une surface de l'implant médical comprend en sus l'anodisation de la surface et la réalisation d'une méthode de dépôt chimique en phase vapeur, le traitement d'une surface entraînant la croissance d'une pluralité de nanotubes de carbone multiparois au sein des lumières d'une pluralité de structures nanotubulaires anodisées.

8. Le procédé de la revendication 7, dans lequel la pluralité de nanotubes de carbone multiparois en combinaison au sein de la pluralité de structures nanotubulaires anodisées constitue le biocapteur qui est attaché à la surface de l'implant médical.

*FIG. 1*

Quartz window

Gas inlet

RF-heated graphite susceptor

Observation port

Sample

Pump out

*FIG. 2*

(a) Unanodized Ti          (b) Anodized nanotubular Ti

(c) Lower magnification          (d) Higher magnification
CNTs grown from the nanotubes of anodized Ti without Co catalyst

(e) Lower magnification          (f) Higher magnification
CNTs grown from the nanotubes of anodized Ti surface with Co catalyst

*FIG. 3*

*FIG. 4*

*FIG. 5*

**(a)**

**(b)**

*FIG. 6*

*FIG. 7*

| Elements | Weight% | Atomic% |
|----------|---------|---------|
| C | 13.16 ± 5.73 | 21.65 |
| O | 45.83 ± 3.09 | 56.62 |
| Mg | 0.20 ± 0.07 | 0.16 |
| P | 13.29 ± 0.91 | 8.48 |
| S | 1.27 ± 0.14 | 0.78 |
| K | 0.29 ± 0.09 | 0.15 |
| Ca | 17.86 ± 1.21 | 8.81 |
| Ti | 8.11 ± 0.58 | 3.35 |
| Co | 0.00 ± 0.00 | 0.00 |

| Elements | Weight% | Atomic% |
|----------|---------|---------|
| C | 64.22 ± 0.70 | 74.79 |
| O | 23.78 ± 0.57 | 20.79 |
| Mg | 0.12 ± 0.3 | 0.07 |
| P | 3.70 ± 0.09 | 1.67 |
| S | 0.06 ± 0.03 | 0.03 |
| K | 0.14 ± 0.02 | 0.05 |
| Ca | 5.62 ± 0.12 | 1.96 |
| Ti | 1.54 ± 0.08 | 0.45 |
| Co | 0.81 ± 0.06 | 0.19 |

*FIG. 8*

24

*FIG. 9*

**FIG. 10**

*FIG. 11*

**FIG. 12**

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006116752 A
- WO 2006004686 A
- WO 2006104644 A
- WO 0187193 A
- WO 2004052447 A
- US 20050038498 A

### Non-patent literature cited in the description

- **OH. S et al.** Significantly accelerated osteoblast cell growth on aligned TiO2 nanotubes. *J Biomed Mater Res A,* 06 April 2001, vol. 78A, 97-103
- **AGER III, J. W. ; BALOOCH, G.** Fracture, aging, and disease in bone. *Journal of Materials Research,* 2006, vol. 21, 1878-92
- **WARD, B. C. ; WEBSTER, T. J.** The effect of nanotopography on calcium and phosphorus deposition on metallic materials, in vitro. *Biomaterials,* 2006, vol. 27, 3064-74
- **LIU, H. ; WEBSTER, T. J.** Nanomedicine for implants: A review of studies and necessary experimental tools. *Biomaterials,* 2007, vol. 28, 354-369
- **OROSZ, GM. ; HANNAN, E. ; MAGAZINER, J. ; KOVAL, K. ; GILBERT, M. ; AUFSES, A. ; STRAUS, E. ; VESPE, E. ; SIU, A. L.** Hip fracture in the older patient: Reasons for delay in hospitalization and timing of surgical repair. *Journal of the American Geriatrics Society,* 2002, vol. 50, 1336-1340
- **YAO, C. ; PERLA, V. ; MCKENZIE, J. L. ; SLAMOVICH, E. B. ; WEBSTER, T. J.** Anodized Ti and Ti6A1V possessing nanometer surface features enhance osteoblast adhesion. *Journal of Biomedical Nanotechnology,* 2005, vol. 1, 68-73
- **ZHU, X. ; CHEN, J. ; SCHEIDELER, L. ; REICHL, R. ; GEIS-GERSTORFER, J.** Effects of topography and composition of titanium surface oxides on osteoblast responses. *Biomaterials,* 2004, vol. 25, 4087-103
- **BEKYAROVA, E. ; NI, Y. ; MALARIKEY, E. B. ; MONTANA, V. ; MCWILLIAMS, J. L. ; HADDON, R. C. ; PARPURA, V.** Applications of carbon nanotubes in biotechnology and biomedicine. *Journal of Biomedical Nanotechnology,* 2005, vol. 1, 3-17
- **KHANG, D. ; SATO, M. ; PRICE, R. L. ; RIBBE, A. E. ; WEBSTER, T. J.** Selective adhesion and mineral deposition by osteoblasts on carbon nanofiber patterns. *International Journal ofNanomedicine,* 2006, vol. 1, 65-72
- **ZANELLO, L.P. ; ZHAO, B. ; HU, H. ; HADDON, R.C.** Bone cell proliferation on carbon nanotubes. *Nano Letters,* 2006, vol. 1, 19-22
- **ZANELLO, L.P.** Electrical properties of osteoblasts cultured on carbon nanotubes. *Micro & Nano Letters,* 2006, vol. 1, 19-22
- **TSUCHIYA, N. ; SATO, Y. ; AOKI, N. ; YOKOYAMA, A. ; WATARI, F. ; MOTOMIYA, K. ; JEYADEVAN, B. ; TOHJI, K.** Evaluation of multi-walled carbon nanotube scaffolds for osteoblast-like cells. *AIP Conference Proceeding,* 2007, vol. 898, 166-169
- **CIOMBOR, D. M. ; AARON, R. K.** Influence of electromagnetic fields on endochondral bone formation. *Journal of Cell Biochemistry,* 1993, vol. 52, 37-41
- **CIOMBOR, D. M. ; AARON, R. K.** The role of electrical stimulation in bone, repair. *Foot Ankle Clinical,* 2005, vol. 10, 579-93
- **CHRISTENSON, E. M. ; ANSETH, K. S. ; VAN DEN BEUCKEN, J. J. J. P. ; CHAN, C. K. ; ERCAN, B. ; JANSEN, J. A. ; LAURENCIN, C. T. ; LI, W. J. ; MURUGAN, R. ; NAIR, L. S.** Nanobiomaterial applications in orthopedics. *Journal of Orthopedic Research,* 2006, vol. 10, 1-12
- **WEBSTER, T. J.** Increased osteoblast adhesion on nanophase metals: Ti, Ti6A14V, and CoCrMo. *Biomaterials,* 2004, vol. 25, 4731-39
- **ZHAO, B. ; HU, H. ; MANDAL, S. K. ; HADDON, R. C.** A bone mimic based on the self-assembly of hydroxyapatite on chemically functionalized single walled carbon nanotubes. *Chemistry of Materials,* 2005, vol. 17, 3235-41
- **SUPRONOWICZ, P. R. ; AJAYAN, P. M. ; ULLMANN, K. R. ; ARULANANDAM, B. P. ; METZGER, D. W. ; BIZIOS, R.** Novel current-conducting composite substrates for exposing osteoblasts to alternating current stimulation. *Journal of Biomedical Materials Research,* 2002, vol. 59, 499-506
- **BESTEMAN, K. ; LEE, J. O. ; WIERTZ, F, G. ; HEERING, H. A. ; DEKKER, C.** Enzyme coated carbon nanotubes as single-molecule biosensors. *Nano Letters,* 2003, vol. 3, 727-30

- **HARRISON, B. S. ; ATALA, A.** Carbon nanotube applications for tissue engineering. *Biomaterial,* 2007, vol. 28, 344-53
- **ROCHEFORT, A. ; AVOURIS, P. ; LESAGE, F. ; SALAHUB, D. R.** Electrical and mechanical properties of distorted carbon nanotubes. *Physical Review B,* 1999, vol. 60, 824-30
- **KREUPL, F. ; GRAHAM, A. P. ; DUESBERG, G. S. ; STEINHÖGL, W. ; LIEBAU, M. ; UNGER, E. ; HÖNLEIN, W.** Carbon nanotubes in interconnect applications. *Microelectronic Engineering,* 2002, vol. 64, 399-408
- **ZHAN, C. ; KACZMAREK, R. ; LOYO-BERRIOS, N. ; SANGL, J. ; BRIGHT, R. A.** *J. Bone Joint Surg. Am.,* 2007, vol. 3, 526-533
- **IIJIMA, S.** *Nature,* 1991, vol. 6348, 56-58
- **LIN, Y. ; TAYLOR, S. ; LI, H. ; FERNANDO, K. A. S. ; QU, L. ; WANG, W. ; GU, L. ; ZHOU, B. ; SUN, Y.** *J. Mater. Chem. Y1,* 2004, vol. 4, 527-541
- **WEBSTER, T. J. ; WAID, M. C. ; MCKENZIE, J. L. ; PRICE, R. L. ; EJIOFOR, J. U.** *Nanotechnology,* 2004, vol. 1, 48-54
- **SMART, S. K. ; CASSADY, A. I. ; LU, G. Q. ; MARTIN, D. J.** *Carbon,* May 2006, vol. 6, 1034-1047
- **ZANELLO, L. P. ; ZHAO, B. ; HU, H. ; HADDON, R. C.** *Nano Lett.,* 2006, vol. 3, 562-567
- **ZANELLO, L. P.** *Micro Nano Lett.,* 2006, vol. 1, 19-22
- **BALANI, K. ; ANDERSON, R. ; LAHA, T. ; ANDARA, M. ; TERCERO, J. ; CRUMPLER, E. ; AGARWAL, A.** *Biomaterials,* February 2007, vol. 4, 618-624
- **CHEN, Y. ; ZHANG, T. H. ; GAN, C. H. ; YU, G.** *Carbon,* vol. 5, 998-1004
- **WEI, W. ; SETHURAMAN, A. ; JIN, C. ; MONTEIRO-RIVIERE, N. A. ; NARAYAN, R. J.** *Journal of Nanoscience and Nanotechnology,* April 2007, 1284-1297
- **HARRISON, B. S. ; ATALA, A.** *Biomaterials,* January 2007, vol. 2, 344-353
- **SUPRONOWICZ, P. R. ; AJAYAN, P. M. ; ULLMANN, K. R. ; ARULANANDAM, B. P. ; METZGER, D. W. ; BIZIOS, R.** *J. Biomed. Mater. Res.,* 2002, vol. 3, 499-506
- **CIOMBOR, D. M. ; AARON, R. K.** *Foot and Ankle Clinics of North America,* December 2005, vol. 4, 579-593
- **AOKI, N. ; YOKOYAMA, A. ; NODASAKA, Y. ; AKASAKA, T. ; UO, M. ; SATO, Y. ; TOHJI, K. ; WATARI, F.** *Journal of Biomedical Nanotechnology,* December 2005, 402-405
- **GIANNONA, S. ; FIRKOWSKA, I. ; ROJAS-CHAPANA, J. ; GIERSIG, M.** *Journal of Nanoscience and Nanotechnology,* April 2007, 1679-1683
- **TSUCHIYA, N. ; SATO, Y. ; AOKI, N. ; YOKOYAMA, A. ; WATARI, F. ; MOTOMIYA, K. ; JEYADEVAN, B. ; TOHJI, K.** Evaluation of Multi-Walled Carbon Nanotube Scaffolds for Osteoblast Growth. AIP, 2007, vol. 898, 166-169
- **SIRIVISOOT, S. ; YAO, C. ; XIAO, X. ; SHELDON, B. W. ; WEBSTER, T. J.** *Nanotechnology,* 2007, vol. 36, 365102
- **PADIGI, S. K. ; REDDY, R. K. K. ; PRASAD, S.** *Biosensors and Bioelectronics,* 15 January 2007, vol. 6, 829-837
- **ROY, S. ; VEDALA, H. ; CHOI, W.** *Nanotechnology,* 2006, vol. 4, S14-S18
- **TANG, H. ; CHEN, J. H. ; HUANG, Z. P. ; WANG, D. Z. ; REN, Z. F. ; NIE, L. H. ; KUANG, Y. F. ; YAO, S. Z.** *Carbon,* 2004, vol. 1, 191-197
- **KURUSU, F. ; KOIDE, S. ; KARUBE, I. ; GOTOH, M.** *Anal. Lett.,* 2006, 903-911
- **LIU, Y. ; WANG, M. ; ZHAO, F. ; XU, Z. ; DONG, S.** *Biosensors and Bioelectronics,* 15 December 2005, vol. 6, 984-988
- **LIU, S. ; LIN, B. ; YANG, X. ; ZHANG, Q. J.** *Phys. Chem. B,* 2007, vol. 5, 1182-1188
- **ROBERTSON, J.** *Material Today,* October 2004, vol. 10, 46-52
- **TALAPATRA, S KAR, S. ; PAL, S. K. ; VAJTAI J. ; CI S. ; VICTOR, P. ; SHAIJUMON, M. M. ; KAUR, S. ; NALAMASU, O. ; AJAYAN, P. M.** *Nat Nano,* 112-116
- **NGO, Q. ; PETRANOVIC, D. ; KRISHNAN, S. ; CASSELL, A. M. ; YE, Q. ; LI, J. ; MEYYAPPAN, M. ; YANG, C. Y.** *Nanotechnology, IEEE Transactions,* 2004, 311-317
- **SATO, S. ; KAWABATA, A. ; KONDO, D. ; NIHEI, M. ; AWANO, Y.** *Chemical Physics Letters,* 24 January 2005, vol. 1-3, 149-154
- **MATHIOWITZ, E.** Encyclopedia of Controlled Drug Delivery. John Wiley & Sons, 1999, vol. 1-2, 1120
- **CALAFIORI, A. ; DI MARCO, G. ; MARTINO, G. ; MAROTTA, M.** *J. Mater. Sci. Mater. Med.*
- **WANG, H. ; LEE, J. ; MOURSI, A. ; LANNUTTI, J. J.** *Journal of Biomedical Materials Research Part A,* 2003, vol. 2, 599-608
- **SOTIROPOULOU, S. ; GAVALAS, V. ; VAMVAKAKI, V. ; CHANIOTAKIS, N. A.** *Biosensors and Bioelectronics,* March 2003, vol. 2-3, 211-215
- **FANG, W. C. ; SUN, C. L. ; HUANG, J. H. ; CHEN, L. C. ; CHYAN, O. ; CHEN, K. H. ; PAPAKONSTANTINOU, P.** *Electrochem. Solid State Lett.,* 2006, vol. 3, A175-A178
- **G.D. CHRISTIAN.** Analytical Chemistry. John Wiley & Sons, 1980
- **ALLEN J. BARD ; LARRY R. FAULKNER.** Electrochemical Methods: Fundamentals and Applications. Wiley, 2000, 856
- **HRAPOVIC, S. ; LIU, Y. ; MALE, K. B. ; LUONG, J. H. T.** *Anal. Chem.,* 2004, vol. 4, 1083-1088

- **HRAPOVIC, S. ; LUONG, J. H. T.** *Anal. Chem.,* 2003, vol. 14, 3308-3315
- **JEANSONNE, B. G. ; FEAGIN, F. F. ; MCMINN, R. W. ; SHOEMAKER, R. L. ; REHM, W. S.** *J. Dent. Res.,* 1979, vol. 4, 1415-1423
- **PUSHPARAJ, V. ; SHAIJUMON, M. ; KUMAR, A. ; MURUGESAN, S. ; CI, L. ; VAJTAI, R. ; LINHARDT, R. ; NALAMASU, O. ; AJAYAN, P.** *Proceedings of the National Academy of Sciences,* 2007, vol. 34, 13574-13577
- **GRAICHEN, F. ; ARNOLD, R. ; ROHLMANN, A. ; BERGMANN, G.** *IEEE Trans. Biomed. Eng.,* 2007, vol. 2, 253-261
- **KAUFMAN, K. ; KOVACEVIC, N. ; IRBY, S. ; COLWELL, C.** *Journal of Biomechanics,* 1996, vol. 5, 667-671
- **D'LIMA, D. ; TOWNSEND, C. ; ARMS, S. ; MORRIS, B. ; COLWELL, C.** *Journal of Biomechanics,* 2005, vol. 2, 299-304
- **BERGMANN, G. ; DEURETZBACHER, G. ; HELLER, M. ; GRAICHEN, F. ; ROHLMANN, A. ; STRAUSS, J. ; DUDA, G. N.** *Journal of Biomechanics,* 2001, vol. 7, 859-871
- **BERGMANN, G. ; GRAICHEN, F. ; ROHLMANN, A. ; VERDONSCHOT, N. ; VAN LENTHE, G. H.** *J. Biomech.,* 2001, vol. 4, 421-428
- **GRAICHEN, F. ; BERGMANN, G. ; ROHLMANN, A.** *Journal of Biomechanics,* 1999, vol. 10, 1113-1117
- **GRAICHEN, F. ; BERGMANN, G.** *Journal of Biomedical Engineering,* 1991, vol. 5, 370-374